# EUROPEAN PATENT SPECIFICATION

(11) **EP 4 045 911 B1**
(45) Date of publication and mention of the grant of the patent: **28.01.2026**
(21) Application number: 20877272.3
(22) Date of filing: 15.10.2020
(51) Int. Cl.: A61K 35/747, G01N 33/569, C12Q 1/689, A61P 15/02, A61P 15/04, A61P 15/06, A61P 15/08, C12N 1/20

(54) **MICROBIOME-BASED INFORMED METHOD TO FORMULATE LIVE BIOTHERAPEUTICS**
MIKROBIOMBASIERTES FUNDIERTES VERFAHREN ZUR HERSTELLUNG VON LEBENDEN BIOTHERAPEUTIKA
PROCÉDÉ INFORMÉ À BASE DE MICROBIOME POUR FORMULER DES AGENTS BIOTHÉRAPEUTIQUES VIVANTS

(30) Priority: 16.10.2019 US 201962915852 P; 10.02.2020 US 202062972243 P; 25.02.2020 US 202016800702; 30.06.2020 US 202016917661
(43) Date of publication of application: 24.08.2022
(73) Proprietor: University of Maryland, Baltimore, Baltimore, MD 21201 (US)
(72) Inventor: RAVEL, Jacques, Laurel, MD 20723 (US); FRANCE, Michael, Baltimore, MD 21201 (US); RUTT, Lindsay, Baltimore, MD 21230 (US); MA, Bing, Baltimore, MD 21201 (US)
(74) Representative: Secerna LLP
(86) International application number: PCT/US2020/055752
(87) International publication number: WO 2021/076740

(56) References cited:
- WO-A1-2019/018348
- BRESHEARS LAURA M. ET AL: "Lactobacillus crispatus inhibits growth of Gardnerella vaginalis and Neisseria gonorrhoeae on a porcine vaginal mucosa model", vol. 15, no. 1, 1 December 2015 (2015-12-01), XP055929614, Retrieved from the Internet <URL:https://bmcmicrobiol.biomedcentral.com/track/pdf/10.1186/s12866-015-0608-0.pdf> DOI: 10.1186/s12866-015-0608-0
- GREEN KATHERINE A ET AL: "Gynecologic health and disease in relation to the microbiome of the female reproductive tract", FERTILITY AND STERILITY, ELSEVIER, AMSTERDAM, NL, vol. 104, no. 6, 24 October 2015 (2015-10-24), pages 1351 - 1357, XP029310658, ISSN: 0015-0282, DOI: 10.1016/J.FERTNSTERT.2015.10.010
- TEIJA OJALA ET AL: "Genome Sequence of Lactobacillus crispatus ST1", JOURNAL OF BACTERIOLOGY, vol. 192, no. 13, 1 July 2010 (2010-07-01), US, pages 3547 - 3548, XP055732199, ISSN: 0021-9193, DOI: 10.1128/JB.00399-10
- MACKLAIM J. M., GLOOR G. B., ANUKAM K. C., CRIBBY S., REID G.: "At the crossroads of vaginal health and disease, the genome sequence of Lactobacillus iners AB-1", PNAS, vol. 108, no. 1, 15 March 2011 (2011-03-15), pages 4688 - 4698, XP055816535
- BOHBOT J.M., DARAÏ E., BRETELLE F., BRAMI G., DANIEL C., CARDOT J.M.: "Efficacy and safety of vaginally administered lyophilized Lactobacillus crispatus IP 174178 in the prevention of bacterial vaginosis recurrence", JOURNAL OF GYNECOLOGY OBSTETRICS AND HUMAN REPRODUCTION, vol. 47, no. 2, February 2018 (2018-02-01), pages 81 - 86, XP055816537
- ITO ET AL.: "Transposon Mutagenesis of Probiotic Lactobacillus casei Identifies asnH, an Asparagine Synthetase Gene Involved in Its Immune-Activating Capacity", PLOS ONE, vol. 9, no. 1, 2014, pages 1 - 10, XP002771087, DOI: 10.1371/journal.pone.0083876
- DATABASE NUCLEOTIDE [online] 27 February 2015 (2015-02-27), ANONYMOUS: "Lactobacillus crispatus ST1 complete genome, strain ST1", XP055699465, retrieved from NCBI Database accession no. FN692037

## Description

### FIELD OF THE INVENTION

The present invention relates generally to a remedial live biotherapeutic formulation for use in medicine.

### BACKGROUND OF THE INVENTION

The microbial communities that inhabit the human body play critical roles in the maintenance of health, and dysfunction of these communities is often associated with disease. Taxonomic profiling of the human microbiome via 16S rRNA gene amplicon sequencing has provided critical insight into the potential role of the microbiota in a wide array of common diseases, including (among others) bacterial vaginosis, Crohn's disease, and psoriasis, but such profiling routinely falls short of describing the etiology of these diseases. This drawback may be due to the fact that, while 16S rRNA gene sequencing can provide species-level taxonomic profiles of a community, it does not describe the genes or metabolic functions that are encoded in the constituents' genomes. This is an important distinction because strains of a bacterial species often exhibit substantial diversity in gene content, such that their genomes harbor sets of accessory genes whose presence is variable. It is therefore difficult, if not impossible, to infer the complete function of a microbial species in an environment using only their 16S rRNA gene sequence. Consequently, to investigate the role of the human microbiome in health and disease, particular emphasis must be placed on describing the gene content and expression of these microbial communities.

Metagenomic and metatranscriptomic profiling are emerging approaches aimed at characterizing the gene content and expression of microbial communities. Results from these approaches have led to increased appreciation for the important role microbial communities play in human health and disease. Despite the rapid development and increased throughput of sequencing technologies, however, current knowledge of the genetic and functional diversity of human microbial communities is still limited. This is due, at least in part, to a lack of resources necessary for the analysis of these massive datasets; *de novo* assembly of metagenomic or metatranscriptomic datasets typically requires substantial computational resources and complicates integration of metagenomic and metatranscriptomic data.

Accurate, high-resolution mapping of metagenomic or metatranscriptomic data against a comprehensive and curated gene database is an alternative analytical strategy that is less computationally demanding, prone to fewer errors, and provides a standard point of reference for comparison of these data. Development of such curated databases is crucial to furthering understanding of the structure and function of microbial communities. In recent years, international initiatives such as MetaHit, the NIH-funded Human Microbiome Project (HMP), and the International Human Microbiome Consortium (IHMC) have been established to generate the resources necessary to enable investigations of the human microbiome, including large reference taxonomic surveys and metagenomic datasets. While multiple 16S rRNA gene catalogs exist, there are relatively few curated resources for referencing metagenomes and metatranscriptomes, and those that do exist focus only on the gut microbiome of either humans or animal model species.

Furthermore, it has been hypothesized that allelic differences in certain genes in the human microbiota may be associated with health or disease, and such allelic differences may thus be useful as selection criteria and/or biomarkers for identification of suitable probiotic strains for use in live biotherapeutic formulations. However, the difficulty of generating gene-specific characterizations of a person's microbiome and a reference gene catalog of a human microbiome have, to date, limited the ability of investigators to discover and exploit such gene-specific health outcomes to formulate live biotherapeutics.

There is thus a need in the art for methods for creating reference gene catalogs for microbiomes of human body sites other than the gut, such as the oral cavity, the skin, and the vagina. There is a further need in the art for methods for formulating and administering live biotherapeutic formulations based on such reference gene catalogs.

### SUMMARY OF THE INVENTION

The present invention is defined in the appended set of claims. In one aspect of the present invention, a remedial live biotherapeutic formulation for use in medicine comprises bacteria adapted to remedy a deficiency or excess of at least one bacterial strain in the subject's vaginal microbial community, and wherein the bacteria comprise a selected strain or consortium of strains comprising at least one of (i) *Lactobacillus crispatus* bacteria configured to express at least one of the asparagine synthase B (*asnB*) gene of SEQ ID NO: 2 and the asparagine synthase B (*asnB*) gene of SEQ ID NO: 3; and (ii) *Lactobacillus crispatus* bacteria containing an asparagine synthase B (*asnB*) gene encoding at least one of a polypeptide of SEQ ID NO: 5 and a polypeptide of SEQ ID NO: 6.

In embodiments, the remedial live biotherapeutic formulation may further comprise a pharmaceutically acceptable carrier.

In embodiments, the remedial live biotherapeutic formulation may further comprise an agent adapted to reduce or remove free ammonia from the vaginal environment.

In embodiments, the remedial live biotherapeutic formulation may be for use in treating bacterial vaginosis, treating pelvic inflammatory disease, treating sexually transmitted infection, treating postpartum endometritis, treating postpartum sepsis, preventing or decreasing the likelihood of preterm birth, preventing or decreasing the likelihood of preterm premature rupture of membranes, preventing or decreasing the likelihood of miscarriage, treating chorioamnionitis, treating intra-amniotic infection, treating infertility, preventing or decreasing the likelihood of ineffectiveness of fertility treatment, treating cervical intraepithelial neoplasia, treating cervical cancer, treating another cancer of the female genitourinary system, decreasing ammonia in the vaginal environment, reducing inflammation, preventing overgrowth of at least one of *Gardnerella vaginalis* and *Prevotella* spp. in the vagina, or a combination thereof.

In embodiments, the remedial live biotherapeutic formulation may further comprise a pharmaceutically acceptable carrier.

In embodiments, the remedial live biotherapeutic formulation may further comprise an agent adapted to reduce or remove free ammonia from the vaginal environment.

In another aspect of the present invention, a live biotherapeutic formulation for use in medicine comprises a selected strain or consortium of strains, wherein the selected strain or consortium of strains consists essentially of any combination of (i) *Lactobacillus crispatus* bacteria configured to express at least one of the asparagine synthase B (*asnB*) gene of SEQ ID NO: 2 and the asparagine synthase B (*asnB*) gene of SEQ ID NO: 3; and (ii) *Lactobacillus crispatus* bacteria containing an asparagine synthase B (*asnB*) gene encoding at least one of a polypeptide of SEQ ID NO: 5 and a polypeptide of SEQ ID NO: 6, wherein the live biotherapeutic formulation is adapted for administration to the vaginal environment of a female human subject .

In embodiments, the live biotherapeutic formulation may further comprise a redox potential control agent.

In embodiments, the live biotherapeutic formulation may further comprise a pH buffer configured to maintain a healthy pH of the vaginal environment.

In embodiments, the live biotherapeutic formulation may further comprise an antimicrobial agent.

In embodiments, the live biotherapeutic formulation may further comprise a growth promoter.

In embodiments, the live biotherapeutic formulation may further comprise a pharmaceutically acceptable carrier.

For purposes of further disclosure and to comply with applicable written description and enablement requirements, the following references generally relate to systems and methods for formulation and administration of live biotherapeutics:
Herman L. Gardner and Charles D. Dukes, "Haemophilus vaginalis vaginitis: a newly defined specific infection previously classified 'nonspecific' vaginitis," 69(5) American Journal of Obstetrics and Gynecology 962 (May 1955) (hereinafter "Gardner").
Sue B. Criswell et al., "Haemophilus vaginalis: vaginitis by inoculation from culture," 33(2) Obstetrics and Gynecology 195 (Feb. 1969) (hereinafter "Criswell").
Richard Amsel et al., "Nonspecific vaginitis: diagnostic criteria and microbial and epidemiologic associations," 74(1) American Journal of Medicine 14 (Jan. 1983) (hereinafter "Amsel").
Helayne M. Silver et al., "Evidence relating bacterial vaginosis to intraamniotic infection," 161(3) American Journal of Obstetrics and Gynecology 808 (Sep. 1989) (hereinafter "Silver").
Robert P. Nugent et al., "Reliability of diagnosing bacterial vaginosis is improved by a standardized method of Gram stain interpretation," 29(2) Journal of Clinical Microbiology 297 (Feb. 1991) (hereinafter "Nugent").
Ronald S. Gibbs, "Chorioamnionitis and bacterial vaginosis," 169(2) American Journal of Obstetrics and Gynecology 460 (Aug. 1993) (hereinafter "Gibbs").
Phillip E. Hay et al., "Abnormal bacterial colonization of the genital tract and subsequent preterm delivery and late miscarriage," 308 BMJ 295 (Jan. 1994) (hereinafter "Hay").
Sharon L. Hillier et al., "Association between bacterial vaginosis and preterm delivery of a low-birth-weight infant," 333(26) New England Journal of Medicine 1737 (Dec. 1995) (hereinafter "Hillier").
J. M. Llahi-Camp et al., "Association of bacterial vaginosis with a history of second trimester miscarriage," 11(7) Human Reproduction 1575 (July 1996) (hereinafter "Llahi-Camp").
Vivien Pybus and Andrew B. Onderdonk, "Evidence for a commensal, symbiotic relationship between Gardnerella vaginalis and Prevotella bivia involving ammonia: potential significance for bacterial vaginosis," 175(2) Journal of Infectious Diseases 406 (Feb. 1997) (hereinafter "Pybus").
T.K. Attwood et al., "The PRINTS protein fingerprint database in its fifth year," 26(1) Nucleic Acids Research 304 (Jan. 1998) (hereinafter "Attwood").
S. G. Ralph et al., "Influence of bacterial vaginosis on conception and miscarriage in the first trimester: cohort study," 319 BMJ 7204 (July 1999) (hereinafter "Ralph").
Virginia S. Ocaña et al., "Characterization of a bacteriocin-like substance produced by a vaginal Lactobacillus salivarius strain," 65(12) Applied and Environmental Microbiology 5631 (Dec. 1999) (hereinafter "Ocaña").
Roman L. Tatusov et al., "The COG database: a tool for genome-scale analysis of protein functions and evolution," 28(1) Nucleic Acids Research 33 (Jan. 2000) (hereinafter "Tatusov").
Gilbert G. Donders et al., "Relationship of bacterial vaginosis and mycoplasmas to the risk of spontaneous abortion," 183(2) American Journal of Obstetrics and Gynecology 431 (Aug. 2000) (hereinafter "Donders").
Janet D. Wilson et al., "Rates of bacterial vaginosis in women undergoing in vitro fertilization for different types of infertility," 109(6) BJOG: an International Journal of Obstetrics and Gynecology 714 (June 2002) (hereinafter "Wilson").
Christian J.A. Sigrist et al., "PROSITE: a documented database using patterns and profiles as motif descriptors," 3(3) Briefings in Bioinformatics 265 (Sep. 2002) (hereinafter "Sigrist").
Daniel H. Haft et al., "The TIGRFAMs database of protein families," 31(1) Nucleic Acids Research 371 (Jan. 2003) (hereinafter "Haft").
Michael J. Ferris et al., "Association of Atopobium vaginae, a recently discovered metronidazole resistant anaerobe, with bacterial vaginosis," 4 BMC Infectious Diseases 5 (Feb. 2004) (hereinafter "Ferris").
Roberta B. Ness et al., "Bacterial vaginosis and risk of pelvic inflammatory disease," 104(4) Obstetrics and Gynecology 761 (Oct. 2004) (hereinafter "Ness I").
Catherine Bru et al., "The ProDom database of protein domain families: more emphasis on 3D," 33(S1) Nucleic Acids Research D212 (Jan. 2005) (hereinafter "Bru").
Roberta B. Ness et al., "A cluster analysis of bacterial vaginosis-associated microflora and pelvic inflammatory disease," 162(6) American Journal of Epidemiology 585 (Sep. 2005) (hereinafter "Ness II").
R. Scott McClelland et al., "Vaginal washing and increased risk of HIV-1 acquisition among African women: a 10-year prospective study," 20(2) AIDS 269 (Jan. 2006) (hereinafter "McClelland I").
Anastasia N. Nikolskaya et al., "PIRSF family classification system for protein functional and evolutionary analysis," 2 Evolutionary Bioinformatics 197 (Jan. 2006) (hereinafter "Nikolskaya").
Catriona S. Bradshaw et al., "High recurrence rates of bacterial vaginosis over the course of 12 months after oral metronidazole therapy and factors associated with recurrence," 193(11) Journal of Infectious Diseases 1478 (Apr. 2006) (hereinafter "Bradshaw I").
Emilia H. Koumans et al., "The prevalence of bacterial vaginosis in the United States, 2001-2004; associations with symptoms, sexual behaviors, and reproductive health," 34(11) Sexually Transmitted Diseases 864 (Nov. 2007) (hereinafter "Koumans").
Miloš Velemínský and Jindřch Tošner, "Relationship of vaginal microflora to PROM, pPROM, and the risk of early-onset neonatal sepsis," 29(2) *Neuroendocrinology Letters* 205 (Apr. 2008) (hereinafter "Velemínský").
Valentina Marcone et al., "Effectiveness of vaginal administration of Lactobacillus rhamnosus following conventional metronidazole therapy: how to lower the rate of bacterial vaginosis recurrences," 31(3) New Microbiologica 429 (July 2008) (hereinafter "Marcone").
Rachel R. Spurbeck and Cindy Grove Arvidson, "Inhibition of Neisseria gonorrhoeae epithelial cell interactions by vaginal Lactobacillus species," 76(7) Infection and Immunity 3124 (July 2008) (hereinafter "Spurbeck").
Katarina Vielfort et al., "Adherence of clinically isolated lactobacilli to human cervical cells in competition with Neisseria gonorrhoeae," 10(12-13) Microbes and Infection 1325 (Oct. 2008) (hereinafter "Vielfort").
Sarah Hunter et al., "InterPro: the integrative protein signature database," 37(S1) Nucleic Acids Research D211 (Jan. 2009) (hereinafter "Hunter").
P. Mastromarino et al., "Effectiveness of Lactobacillus-containing vaginal tablets in the treatment of symptomatic bacterial vaginosis," 15(1) Clinical Microbiology and Infection 67 (Jan. 2009) (hereinafter "Mastromarino").
Bart Ferwerda et al., "Human dectin-1 deficiency and mucocutaneous fungal infections," 361 New England Journal of Medicine 1760 (Oct. 2009) (hereinafter "Ferwerda").
David M. Tanenbaum et al., "The JCVI standard operating procedure for annotating prokaryotic metagenomic shotgun sequencing data," 2 Standards in Genomic Sciences 229 (Apr. 2010) (hereinafter "Tanenbaum").
Jeanne M. Marrazzo et al., "Risks for acquisition of bacterial vaginosis among women who report sex with women: a cohort study," 5(6) PLOS One e11139 (June 2010) (hereinafter "Marrazzo").
PCT Application Publication 2010/079991, entitled "Vector for treatment vaccine for stable and constitutive high-expression cervical cancer and recombinant Lactobacillus transformed by the same," published 18 November 2010 to Sung et al. (hereinafter "Sung").
Charles A. Rivers et al., "Prevalence of bacterial vaginosis and vulvovaginal candidiasis mixed infection in a southeastern American STD clinic," 38(7) Sexually Transmitted Diseases 672 (July 2011) (hereinafter "Rivers").
Maria Trent et al., "Recurrent PID, subsequent STI, and reproductive health outcomes: findings from the PID Evaluation and Clinical Health (PEACH) study," 38(9) Sexually Transmitted Diseases 879 (Sep. 2011) (hereinafter "Trent").
Benjamin M. Ngugi et al., "Effects of BV-associated bacteria and sexual intercourse on vaginal colonization with the probiotic Lactobacillus crispatus CTV-05," 38(11) Sexually Transmitted Diseases 1020 (Nov. 2011) (hereinafter "Ngugi").
Minoru Kanehisa et al., "KEGG for integration and interpretation of large-scale molecular data sets," 40(D1) Nucleic Acids Research D109 (Jan. 2012) (hereinafter "Kanehisa").
Richard W. Hyman et al., "The dynamics of the vaginal microbiome during infertility therapy with in vitro fertilization-embryo transfer," 29(2) Journal of Assisted Reproduction and Genetics 105 (Feb. 2012) (hereinafter "Hyman I").
Catriona S. Bradshaw et al., "Efficacy of oral metronidazole with vaginal clindamycin or vaginal probiotic for bacterial vaginosis: randomized placebo-controlled double-blind trial," 7(4) PLOS One e34540 (Apr. 2012) (hereinafter "Bradshaw II").
Kjersti Aagaard et al., "A metagenomic approach to characterization of the vaginal microbiome signature in pregnancy," 7(6) PLOS One e36466 (June 2012) (hereinafter "Aagaard").
Sujatha Srinivasan et al., "Bacterial communities in women with bacterial vaginosis: high resolution phylogenetic analyses reveal relationships of microbiota to clinical criteria," 7(6) PLOS One e37818 (June 2012) (hereinafter "Srinivasan").
Caroline M. Mitchell et al., "Effect of sexual debut on vaginal microbiota in a cohort of young women," 120(6) Obstetrics and Gynecology 1306 (Dec. 2012) (hereinafter "Mitchell").
Catriona S. Bradshaw et al., "Prevalent and incident bacterial vaginosis are associated with sexual and contraceptive behaviors in young Australian women," 8(3) PLOS One e57688 (Mar. 2013) (hereinafter "Bradshaw III").
Elena Shipitsyna et al., "Composition of the vaginal microbiota in women of reproductive age-sensitive and specific molecular diagnosis of bacterial vaginosis is possible?", 8(4) PLOS One e60670 (Apr. 2013) (hereinafter "Shipitsyna").
Aziz Homayouni et al., "Effects of probiotics on the recurrence of bacterial vaginosis: a review," 18(1) Journal of Lower Genital Tract Disease 79 (Jan. 2014) (hereinafter "Homayouni").
Richard W. Hyman et al., "Diversity of the vaginal microbiome correlates with preterm birth," 21(1) Reproductive Sciences 32 (Jan. 2014) (hereinafter "Hyman II").
Harsha Sharma et al., "Microbiota and pelvic inflammatory disease," 32(1) Seminars in Reproductive Medicine 43 (Jan. 2014) (hereinafter "Sharma").
Ido Sirota et al., "Potential influence of the microbiome on infertility and assisted reproductive technology," 32(1) Seminars in Reproductive Medicine 35 (Jan. 2014) (hereinafter "Sirota").
Roberto Romero et al., "The composition and stability of the vaginal microbiota of normal pregnant women is different from that of non-pregnant women," 2(1) Microbiome 4 (Feb. 2014) (hereinafter "Romero I").
Roberto Romero et al., "The vaginal microbiota of pregnant women who subsequently have spontaneous preterm labor and delivery and those with a normal delivery at term," 2 Microbiome 18 (May 2014) (hereinafter "Romero II").
Luca Laghi et al., "Rifaximin modulates the vaginal microbiome and metabolome in women affected by bacterial vaginosis," 58(6) Antimicrobial Agents and Chemotherapy 3411 (June 2014) (hereinafter "Laghi").
Galina Stoyancheva et al., "Bacteriocin production and gene sequencing analysis from vaginal Lactobacillus strains," 196(9) Archives of Microbiology 645 (June 2014) (hereinafter "Stoyancheva").
Marina R. S. Walther-António, "Pregnancy's stronghold on the vaginal microbiome," 9(6) PLOS One e98514 (June 2014) (hereinafter "Walther-António").
U.S. Patent 8,846,027, entitled "Compositions for the vaginal and oral administration of Lactobacillus and uses thereof," issued 30 September 2014 to Kiss et al. (hereinafter "Kiss").
Roxana J. Hickey and Larry J. Forney, "Gardnerella vaginalis does not always cause bacterial vaginosis," 210(10) Journal of Infectious Diseases 1682 (Nov. 2014) (hereinafter "Hickey I").
Teija Ojala et al., "Comparative genomics of Lactobacillus crispatus suggest novel mechanisms for the competitive exclusion of Gardnerella vaginalis," 15 BMC Genomics 1070 (Dec. 2014) (hereinafter "Ojala").
Ivica Letunic et al., "SMART: recent updates, new developments and status in 2015," 43(D1) Nucleic Acids Research D257 (Jan. 2015) (hereinafter "Letunic").
Ivo Pedruzzi et al., "HAMAP in 2015: updates to the protein family classification and annotation system," 43(D1) Nucleic Acids Research D1064 (Jan. 2015) (hereinafter "Pedruzzi").
Emilio Potenza et al., "MobiDB 2.0: an improved database of intrinsically disordered and mobile proteins," 43(D1) Nucleic Acids Research D315 (Jan. 2015) (hereinafter "Potenza").
Roxana J. Hickey et al., "Vaginal microbiota of adolescent girls prior to the onset of menarche resemble those of reproductive-age women," 6(2) mBio e00097 (Mar. 2015) (hereinafter "Hickey II").
Lenka A. Vodstrcil et al., "Incident bacterial vaginosis (BV) in women who have sex with women is associated with behaviors that suggest sexual transmission of BV," 60(7) Clinical Infectious Diseases 1042 (Apr. 2015) (hereinafter "Vodstrcil").
Linnet Masese et al., "Changes in the contribution of genital tract infections to HIV acquisition among Kenyan high-risk women from 1993 to 2012," 29(9) AIDS 1077 (June 2015) (hereinafter "Masese").
Daniel B. DiGiulio et al., "Temporal and spatial variation of the human microbiota during pregnancy," 112(35) Proceedings of the National Academy of Sciences 11060 (Sep. 2015) (hereinafter "DiGiulio").
PCT Application Publication 2015/173693, entitled "Compositions containing boric acid and a mixture of Lactobacillus," published 19 November 2015 to de Seta et al. (hereinafter "de Seta").
Antonietta Rizzo et al., "Lactobacillus crispatus mediates anti-inflammatory cytokine interleukin-10 induction in response to Chlamydia trachomatis infection in vitro," 305(8) International Journal of Medical Microbiology 815 (Dec. 2015) (hereinafter "Rizzo").
Robert D. Finn et al., "The Pfam protein families database: towards a more sustainable future," 44(D1) Nucleic Acids Research D279 (Jan. 2016) (hereinafter "Finn").
Jaime Huerta-Cepas et al., "eggNOG 4.5: a hierarchical orthology framework with improved functional annotations for eukaryotic, prokaryotic and viral sequences," 44(D1) Nucleic Acids Research D286 (Jan. 2016) (hereinafter "Huerta-Cepas").
Su Datt Lam et al., "Gene3D: expanding the utility of domain assignments," 44(D1) Nucleic Acids Research D404 (Jan. 2016) (hereinafter "Lam").
Elahe Motevaseli et al., "The effect of Lactobacillus crispatus and Lactobacillus rhamnosus culture supernatants on expression of autophagy genes and HPV E6 and E7 oncogenes in the HeLa cell line," 17(4) Cell Journal 601 (Jan. 2016) (hereinafter "Motevaseli").
T. Haahr et al., "Abnormal vaginal microbiota may be associated with poor reproductive outcomes: a prospective study in IVF patients," 31(4) Human Reproduction 795 (Apr. 2016) (hereinafter "Haahr I").
Joke A. M. Dols et al., "Molecular assessment of bacterial vaginosis by Lactobacillus abundance and species diversity," 16 BMC Infectious Diseases 180 (Apr. 2016) (hereinafter "Dols").
Xiaowei Sun et al., "Modulating the human microbiome with live biotherapeutic products: intellectual property landscape," 15(4) Nature Reviews Drug Discovery 224 (Apr. 2016) (hereinafter "Sun").
Gary Ventolini, "Progresses in vaginal microflora physiology and implications for bacterial vaginosis and candidiasis," 12(3) Women's Health 283 (May 2016) (hereinafter "Ventolini").
Paola Nardini et al., "Lactobacillus crispatus inhibits the infectivity of Chlamydia trachomatis elementary bodies, in vitro study," 6 Scientific Reports 29024 (June 2016) (hereinafter "Nardini").
C. Genovese et al., "Alterations of the vaginal microbiota in the third trimester of pregnancy and pPROM," 20(16) European Review for Medical and Pharmacological Sciences 3336 (Aug. 2016) (hereinafter "Genovese").
PCT Application Publication 2016/121865, entitled "Lactic-acid-bacteria-containing composition, oral pharmaceutical composition for treating HPV infection and/or HPV-associated tumors, and mucosal immunity-inducing agent," published 4 August 2016 to Kawana et al. (hereinafter "Kawana").
Lindsay M. Kindinger et al., "Relationship between vaginal microbiota dysbiosis, inflammation, and pregnancy outcomes in cervical cerclage," 8(350) Science Translational Medicine 350ra102 (Aug. 2016) (hereinafter "Kindinger").
Catherine L. Haggerty et al., "Identification of novel microbes associated with pelvic inflammatory disease and infertility," 92(6) Sexually Transmitted Infections 441 (Sep. 2016) (hereinafter "Haggerty").
Ki Ho Hong et al., "Analysis of the vaginal microbiome by next-generation sequencing and evaluation of its performance as a clinical diagnostic tool in vaginitis," 36(5) Annals of Laboratory Medicine 441 (Sep. 2016) (hereinafter "Hong").
Teenus Paramel Jayaprakash et al., "High diversity and variability in the vaginal microbiome in women following preterm premature rupture of membranes (PPROM): a prospective cohort study," 11(11) PLOS One e0166794 (Nov. 2016) (hereinafter "Paramel Jayaprakash").
Charlotte van der Veer et al., "The cervicovaginal microbiota in women notified for Chlamydia trachomatis infection: a case-control study at the sexually transmitted infection outpatient clinic in Amsterdam, the Netherlands," 64(1) Clinical Infectious Diseases 24 (Jan. 2017) (hereinafter "van der Veer").
Rogers A. Ñahui Palomino et al., "Vaginal Lactobacillus inhibits HIV-1 replication in human tissues ex vivo," 8 Frontiers in Microbiology 906 (May 2017) (hereinafter "Ñahui Palomino").
Thor Haahr et al., "Treatment of abnormal vaginal microbiota before frozen embryo transfer: case-report and minireview to discuss the longitudinal treatment efficacy of oral clindamycin," 8 Frontiers in Physiology 415 (June 2017) (hereinafter "Haahr II").
Immaculada Moreno and Jason M. Franasiak, "Endometrial microbiota-new player in town," 108(1) Fertility and Sterility 32 (July 2017) (hereinafter "Moreno").
Giuseppina Campisciano et al., "Subclinical alteration of the cervical-vaginal microbiome in women with idiopathic infertility," 232(7) Journal of Cellular Physiology 1681 (July 2017) (hereinafter "Campisciano").
Benjamin J. Callahan et al., "Replication and refinement of a vaginal microbial signature of preterm birth in two racially distinct cohorts of US women," 114(37) Proceedings of the National Academy of Sciences 9966 (Sep. 2017) (hereinafter "Callahan").
B. Shannon et al., "Association of HPV infection and clearance with cervicovaginal immunology and the vaginal microbiota," 10(5) Mucosal Immunology 1310 (Sep. 2017) (hereinafter "Shannon").
Molly J. Stout et al., "Early pregnancy vaginal microbiome trends and preterm birth," 217(3) American Journal of Obstetrics and Gynecology 356.e1 (Sep. 2017) (hereinafter "Stout").
Richard G. Brown et al., "Vaginal dysbiosis increases risk of preterm fetal membrane rupture, neonatal sepsis and is exacerbated by erythromycin," 16 BMC Medicine 9 (Jan. 2018) (hereinafter "Brown").
Erica L. Plummer et al., "Combined oral and topical antimicrobial therapy for male partners of women with bacterial vaginosis: acceptability, tolerability and impact on the genital microbiota of the couples-a pilot study," 13(1) PLOS One e0190199 (Jan. 2018) (hereinafter "Plummer").
Noa Ziklo et al., "Dysbiosis of the vaginal microbiota and higher vaginal kynurenine/tryptophan ratio reveals an association with Chlamydia trachomatis genital infections," 8 Frontiers in Cellular and Infection Microbiology 1 (Jan. 2018) (hereinafter "Ziklo").
U.S. Patent 9,896,733, entitled "Method and kit for prediction success of in vitro fertilization," issued 20 February 2018 to Kok et al. (hereinafter "Kok").
Robin van Houdt et al., "Lactobacillus iners-dominated vaginal microbiota is associated with increased susceptibility to Chlamydia trachomatis infection in Dutch women, a case control study," 94(2) Sexually Transmitted Infections 117 (Mar. 2018) (hereinafter "van Houdt").
Paul Nyirjesy and Jane R. Schwebke, "Secnidazole: next-generation antimicrobial agent for bacterial vaginosis treatment," 13(5) Future Microbiology 507 (Apr. 2018) (hereinafter "Nyirjesy").
Steven E. Chavoustie et al., "A phase 3, multicenter, prospective, open-label study to evaluate the safety of a single dose of secnidazole 2 g for the treatment of women and postmenarchal adolescent girls with bacterial vaginosis," 27(4) Journal of Women's Health 492 (Apr. 2018) (hereinafter "Chavoustie").
U.S. Patent Application Publication 2018/0114592, entitled "Method and system for characterizing allergy-related conditions associated with microorganisms," published 26 April 2018 to Apte et al. (hereinafter "Apte I").
R. Scott McClelland et al., "Evaluation of the association between the concentrations of key vaginal bacteria and the increased risk of HIV acquisition in African women from five cohorts: a nested case-control study," 18(5) Lancet Infectious Diseases 554 (May 2018) (hereinafter "McClelland II").
Xi Yang et al., "Role of Lactobacillus in cervical cancer," 2018(10) Cancer Management and Research 1219 (May 2018) (hereinafter "Yang").
Bryan A. Wee et al., "A retrospective pilot study to determine whether the reproductive tract microbiota differs between women with a history of infertility and fertile women," 58(3) Australian and New Zealand Journal of Obstetrics and Gynecology 341 (June 2018) (hereinafter "Wee").
Samuel J. Kroon et al., "Cervicovaginal microbiota, women's health, and reproductive outcomes," 110(3) Fertility and Sterility 327 (Aug. 2018) (hereinafter "Kroon").
Wojciech Kwasniewski et al., "Microbiota dysbiosis is associated with HPV-induced cervical carcinogenesis," 16(6) Oncology Letters 7035 (Dec. 2018) (hereinafter "Kwasniewski").
Jeanne Tamarelle et al., "Vaginal microbiota composition and association with prevalent Chlamydia trachomatis infection: a cross-sectional study of young women attending a STI clinic in France," 94(8) Sexually Transmitted Infections 616 (Dec. 2018) (hereinafter "Tamarelle").
U.S. Patent 10,169,541, entitled "Method and system for characterizing skin related conditions," issued 1 January 2019 to Apte et al. (hereinafter "Apte II").
U.S. Patent 10,246,753, entitled "Method and system for characterizing mouth-associated conditions," issued 2 April 2019 to Apte et al. (hereinafter "Apte III").
J. Norenhag et al., "The vaginal microbiota, human papillomavirus and cervical dysplasia: a systematic review and network meta-analysis," 127(2) BJOG 171 (Jan. 2020) (hereinafter "Norenhag").
Kyeong A. So et al., "Changes of vaginal microbiota during cervical carcinogenesis in women with human papillomavirus infection," 15(9) PLoS One e0238705 (Sep. 2020) (hereinafter "So").

As used herein, "at least one," "one or more," and "and/or" are open-ended expressions that are both conjunctive and disjunctive in operation. For example, each of the expressions "at least one of A, B, and C," "at least one of A, B, or C," "one or more of A, B, and C," "one or more of A, B, or C," and "A, B, and/or C" means A alone, B alone, C alone, A and B together, A and C together, B and C together, or A, B, and C together.

It is to be noted that the term "a" or "an" entity refers to one or more of that entity. As such, the terms "a" (or "an"), "one or more," and "at least one" can be used interchangeably herein. It is also to be noted that the terms "comprising," "including," and "having" can be used interchangeably.

The embodiments and configurations described herein are neither complete nor exhaustive. As will be appreciated, other embodiments of the invention are possible utilizing, alone or in combination, one or more of the features set forth above or described in detail below.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.
Figure 1 is a flowchart illustrating a data processing and integration scheme for the construction of a human vaginal integrated non-redundant gene catalog (VIRGO).
Figure 2 is a graph of the percentage of vaginal metagenome reads that can be mapped to contigs from various reference data sets.
Figure 3A is a set of species-specific metagenome accumulation curves for the number of non-redundant genes.
Figure 3B is a graph of the functional distribution of non-redundant genes in VIRGO.
Figure 3C is a boxplot of the twenty species having the most abundant gene content in VIRGO.
Figure 4A is a boxplot of the number of non-redundant genes in samples of different community state types (CSTs).
Figure 4B is a plot of the log₂ transformed ratio of the abundance of genes of a species in high gene count (HGC) communities to the same abundance in low gene count (LGC) communities.
Figure 5A is an illustration of an analysis of a female human subject's vaginal metagenomes and associated metatranscriptomes were analyzed at four time points: prior to (T1), during (T2 and T3), and after (T4) an episode of symptomatic bacterial vaginosis.
Figure 5B is a functional profile of the metagenome (MG) and metatranscriptome (MT) of Figure 5A at each of the four time points.
Figure 5C is a functional profile of the metagenome and metatranscriptome of Figure 5A at each of the four time points, stratified by species using the taxonomic profiling provided by VIRGO.
Figure 6 is an illustration of a relationship between the depth of sequencing and the number of non-redundant genes identified using VIRGO.
Figures 7A and 7B are heatmaps of the presence or absence of non-redundant gene profiles of *L. crispatus* and *L. gasseri,* respectively, for 56 available isolate genomes (gray) and 413 VIRGO-characterized metagenomes that contained either more than 50% relative species abundance (red: *L. crispatus;* green: *L. gasseri)* or less than 50% relative species abundance (cyan).
Figure 8 is a maximum likelihood tree of the *L. crispatus* asparagine synthase B (asnB) gene.

### DETAILED DESCRIPTION OF THE INVENTION

Unless defined otherwise, all technical and scientific terms used herein have the same meaning as is commonly understood by one of ordinary skill in the art.. In the event that there is a plurality of definitions for a term herein, the definition provided in the Brief Summary of the Invention prevails unless otherwise stated.

"CRISPR" (Clustered Regularly Interspaced Short Palindromic Repeats) loci refers to certain genetic loci encoding components of DNA cleavage systems, for example, used by bacterial and archaeal cells to destroy foreign DNA. A CRISPR locus can consist of a CRISPR array, comprising short direct repeats (CRISPR repeats) separated by short variable DNA sequences (called spacers), which can be flanked by diverse Cas (CRISPR-associated) genes. The *CRISPR-*Cas system, an example of a pathway that was unknown to science prior to the DNA sequencing era, is now understood to confer bacteria and archaea with acquired immunity against phage and viruses. Intensive research over the past decade has uncovered the biochemistry of this system. *CRISPR-*Cas systems consist of Cas proteins, which are involved in acquisition, targeting and cleavage of foreign DNA or RNA, and a *CRISPR* array, which includes direct repeats flanking short spacer sequences that guide Cas proteins to their targets. Class 2 *CRISPR-*Cas are streamlined versions in which a single Cas protein bound to RNA is responsible for binding to and cleavage of a targeted sequence. The programmable nature of these minimal systems has facilitated their use as a versatile technology that is revolutionizing the field of genome manipulation.

As used herein, an "effector" or "effector protein" is a protein that encompasses an activity including recognizing, binding to, and/or cleaving or nicking a polynucleotide target. An effector, or effector protein, may also be an endonuclease. The "effector complex" of a CRISPR system includes Cas proteins involved in crRNA and target recognition and binding. Some of the component Cas proteins may additionally comprise domains involved in target polynucleotide cleavage.

The term "Cas protein" refers to a polypeptide encoded by a Cas (CRISPR-associated) gene. A Cas protein includes proteins encoded by a gene in a Cas locus, and include adaptation molecules as well as interference molecules. An interference molecule of a bacterial adaptive immunity complex includes endonucleases. A Cas endonuclease described herein comprises one or more nuclease domains. A Cas endonuclease includes but is not limited to: the novel Cas-alpha protein disclosed herein, a Cas9 protein, a Cpf1 (Cas12) protein, a C2c1 protein, a C2c2 protein, a C2c3 protein, Cas3, Cas3-HD, Cas 5, Cas7, Cas8, Cas10, or combinations or complexes of these. A Cas protein may be a "Cas endonuclease" or "Cas effector protein", that when in complex with a suitable polynucleotide component, is capable of recognizing, binding to, and optionally nicking or cleaving all or part of a specific polynucleotide target sequence.

CRISPR-Cas systems have been classified according to sequence and structural analysis of components. Multiple CRISPR/Cas systems have been described including Class 1 systems, with multisubunit effector complexes (comprising type I, type III, and type IV), and Class 2 systems, with single protein effectors (comprising type II, type V, and type VI). A CRISPR-Cas system comprises, at a minimum, a CRISPR RNA (crRNA) molecule and at least one CRISPR-associated (Cas) protein to form crRNA ribonucleoprotein (crRNP) effector complexes. CRISPR-Cas loci comprise an array of identical repeats interspersed with DNA-targeting spacers that encode the crRNA components and an operon-like unit of Cas genes encoding the Cas protein components. The resulting ribonucleoprotein complex recognizes a polynucleotide in a sequence-specific manner. The crRNA serves as a guide RNA for sequence specific binding of the effector (protein or complex) to double strand DNA sequences, by forming base pairs with the complementary DNA strand while displacing the noncomplementary strand to form a so called R-loop. RNA transcripts of CRISPR loci (pre-crRNA) are cleaved specifically in the repeat sequences by CRISPR associated (Cas) endoribonucleases in type I and type III systems or by RNase III in type II systems. The number of CRISPR-associated genes at a given CRISPR locus can vary between species.

Different Cas genes that encode proteins with different domains are present in different CRISPR systems. The Cas operon comprises genes that encode for one or more effector endonucleases, as well as other Cas proteins. Some domains may serve more than one purpose, for example Cas9 comprises domains for endonuclease functionality as well as for target cleavage, among others. The Cas endonuclease is guided by a single CRISPR RNA (crRNA) through direct RNA-DNA base-pairing to recognize a DNA target site that is in close vicinity to a protospacer adjacent motif (PAM). Class I CRISPR-Cas systems comprise Types I, III, and IV. A characteristic feature of Class I systems is the presence of an effector endonuclease complex instead of a single protein. A Cascade complex comprises an RNA recognition motif (RRM) and a nucleic acid-binding domain that is the core fold of the diverse RAMP (Repeat-Associated Mysterious Proteins) protein superfamily.

Type I CRISPR-Cas systems comprise a complex of effector proteins, termed Cascade (CRISPR-associated complex for antiviral defense) comprising at a minimum Cas5 and Cas7. The effector complex functions together with a single CRISPR RNA (crRNA) and Cas3 to defend against invading viral DNA. Type I systems are divided into seven subtypes.

Type III CRISPR-Cas systems, comprising a plurality of cas7 genes, target either ssRNA or ssDNA, and function as either an RNase as well as a target RNA-activated DNA nuclease. Type IV systems, although comprising typical type I cas5 and cas7 domains in addition to a cas8-like domain, may lack the CRISPR array that is characteristic of most other CRISPR-Cas systems.

Class II CRISPR-Cas systems comprise Types II, V, and VI. A characteristic feature of Class II systems is the presence of a single Cas effector protein instead of an effector complex. Types II and V Cas proteins comprise an RuvC endonuclease domain that adopts the RNase H fold. Type II CRISPR/Cas systems employ a crRNA and tracrRNA (trans-activating CRISPR RNA) to guide the Cas endonuclease to its DNA target. The crRNA comprises a spacer region complementary to one strand of the double strand DNA target and a region that base pairs with the tracrRNA (trans-activating CRISPR RNA) forming a RNA duplex that directs the Cas endonuclease to cleave the DNA target, leaving a blunt end. Spacers are acquired through a not fully understood process involving Cas1 and Cas2 proteins. Type II CRISPR/Cas loci typically comprise cas1 and cas2 genes in addition to the cas9 gene. Type II CRISR-Cas loci can encode a tracrRNA, which is partially complementary to the repeats within the respective CRISPR array and can comprise other proteins such as Csn1 and Csn2. The presence of cas9 in the vicinity of cas1 and cas2 genes is the hallmark of type II loci. Type V CRISPR/Cas systems comprise a single Cas endonuclease, including Cpf1 (Cas12) that is an active RNA-guided endonuclease that does not necessarily require the additional trans-activating CRISPR (tracr) RNA for target cleavage, unlike Cas9. Type VI CRISPR-Cas systems comprise a *cas13* gene that encodes a nuclease with two HEPN (Higher Eukaryotes and Prokaryotes Nucleotide-binding) domains but no HNH or RuvC domains and are not dependent upon tracrRNA activity. The majority of HEPN domains comprise conserved motifs that constitute a metal-independent endoRNase active site. Because of this feature, it is thought that type VI systems act on RNA targets instead of the DNA targets that are common to other CRISPR-Cas systems.

Disclosed herein but not claimed is a method for ameliorating, treating, or preventing a malignancy in a female human subject, comprising (a) generating a gene-specific characterization, at an intraspecies level, of the subject's vaginal microbial community; (b) comparing the gene-specific characterization to a reference gene catalog of the human vaginal microbiome, wherein the reference gene catalog comprises at least one metagenome or single-strain genome associated with a healthy microbiome; (c) identifying, based on the comparison of step (b), a deficiency or excess of at least one bacterial strain in the subject's vaginal microbial community; (d) formulating a remedial live biotherapeutic formulation, comprising bacteria adapted to remedy the deficiency or excess of the at least one bacterial strain in the subject's vaginal microbial community; and (e) administering the remedial live biotherapeutic formulation to the subject.

The malignancy may be a cancer of the female genitourinary system.

The bacteria adapted to remedy the deficiency or excess of the at least one bacterial strain in the subject's vaginal microbial community may comprise a selected strain or consortium of strains of a bacterial species selected from the group consisting of *Lactobacillus crispatus, Lactobacillus gasseri,* and *Lactobacillus jensenii.* The bacteria may, but need not, comprise at least one of (i) *Lactobacillus crispatus* bacteria configured to express, carry, harbor, or encode at least one of the asparagine synthase B (asnB) gene of SEQ ID NO: 2 and the asparagine synthase B (asnB) gene of SEQ ID NO: 3; and (ii) *Lactobacillus crispatus* bacteria containing an asparagine synthase B (asnB) gene encoding at least one of a polypeptide of SEQ ID NO: 5 and a polypeptide of SEQ ID NO: 6.

Step (a) may comprise at least one sub-step selected from the group consisting of (i) preprocessing sequence data for each of one or more samples by at least one of removing human contaminants from a sample, quality-filtering, and removing ribosomal RNA; (ii) assembling at least one metagenome from each of one or more samples by executing a procedure to generate one or more nucleotide based de novo assemblies; (iii) compiling coding DNA sequences (CDSs); (iv) applying at least one taxonomic annotation by transitive assignment of species name from reads mapping to contigs; and (v) applying at least one functional annotation using annotations from one or more functional databases.

The live biotherapeutic formulation may further comprise a pharmaceutically acceptable carrier.

The live biotherapeutic formulation may further comprise an agent adapted to reduce or remove free ammonia from the vaginal environment.

Also disclosed herein but not claimed is a method for achieving an improvement in the vaginal health of a female human subject, comprising (a) generating a gene-specific characterization, at an intraspecies level, of the subject's vaginal microbial community; (b) comparing the gene-specific characterization to a reference gene catalog of the human vaginal microbiome, wherein the reference gene catalog comprises at least one metagenome or single-strain genome associated with a healthy microbiome; (c) identifying, based on the comparison of step (b), a deficiency or excess of at least one bacterial strain in the subject's vaginal microbial community; (d) formulating a remedial live biotherapeutic formulation, comprising bacteria adapted to remedy the deficiency or excess of the at least one bacterial strain in the subject's vaginal microbial community; and (e) administering the remedial live biotherapeutic formulation to the subject, wherein the improvement is selected from the group consisting of treating bacterial vaginosis in the subject, decreasing ammonia in the vaginal environment of the subject, reducing inflammation in the subject, preventing overgrowth of at least one of *Gardnerella vaginalis* and *Prevotella* spp. in the vagina of the subject, and combinations thereof.

The bacteria adapted to remedy the deficiency or excess of the at least one bacterial strain in the subject's vaginal microbial community may comprise a selected strain or consortium of strains of a bacterial species selected from the group consisting of *Lactobacillus crispatus, Lactobacillus gasseri,* and *Lactobacillus jensenii.* The bacteria may, but need not, comprise at least one of (i) *Lactobacillus crispatus* bacteria configured to express at least one of the asparagine synthase B (asnB) gene of SEQ ID NO: 2 and the asparagine synthase B (asnB) gene of SEQ ID NO: 3; and (ii) *Lactobacillus crispatus* bacteria containing an asparagine synthase B (asnB) gene encoding at least one of a polypeptide of SEQ ID NO: 5 and a polypeptide of SEQ ID NO: 6. The bacteria may, but need not, comprise *Lactobacillus crispatus,* and the preselected strain or consortium of strains may, but need not, comprise at least one strain selected from the group consisting of LUCA111, LUCA011, LUCA015, LUCA009, LUCA102, LUCA006, LUCA059, LUCA103, and LUCA008.

Step (a) may comprise at least one sub-step selected from the group consisting of (i) preprocessing sequence data for each of one or more samples by at least one of removing human contaminants from a sample, quality-filtering, and removing ribosomal RNA; (ii) assembling at least one metagenome from each of one or more samples by executing a procedure to generate one or more nucleotide based de novo assemblies; (iii) compiling coding DNA sequences (CDSs); (iv) applying at least one taxonomic annotation by transitive assignment of species name from reads mapping to contigs; and (v) applying at least one functional annotation using annotations from one or more functional databases.

The live biotherapeutic formulation may further comprise a pharmaceutically acceptable carrier.

The live biotherapeutic formulation further may further comprise an agent adapted to reduce or remove free ammonia from the vaginal environment.

It is one aspect of the present invention to provide a live biotherapeutic formulation adapted for administration to the vaginal environment of a female human subject, comprising one or more of (i) *Lactobacillus crispatus* bacteria configured to express at least one of the asparagine synthase B (asnB) gene of SEQ ID NO: 2 and the asparagine synthase B (asnB) gene of SEQ ID NO: 3; and (ii) *Lactobacillus crispatus* bacteria containing an asparagine synthase B (asnB) gene encoding at least one of a polypeptide of SEQ ID NO: 5 and a polypeptide of SEQ ID NO: 6.

In embodiments, the live biotherapeutic formulation may further comprise a redox potential control agent.

In embodiments, the live biotherapeutic formulation may further comprise a pH buffer configured to maintain a healthy pH of the vaginal environment.

In embodiments, the live biotherapeutic formulation may further comprise an antimicrobial agent.

In embodiments, the live biotherapeutic formulation may further comprise a growth promoter.

In embodiments, the live biotherapeutic formulation may further comprise a pharmaceutically acceptable carrier.

Also disclosed herein but not claimed are methods and systems for constructing reference gene catalogs for human microbiomes, i.e. integrated and comprehensive resources to establish taxonomic and functional profiling of microbiomes, e.g. vaginal microbiomes, from metagenomic and metatransciptomic datasets. In the case of vaginal microbiomes specifically, such reference gene catalogs can be constructed using a combination of metagenomes and urogenital bacterial isolate genomes. The genes identified in these data can be further clustered into vaginal orthologous groups (VOGs), providing a catalog of functional protein families common to vaginal microbiomes. The gene catalog can be curated with taxonomic assignments as well as functional features using diverse protein databases. Importantly, the present inventors have shown that reference gene catalogs constructed according to the present disclosure can provide greater than 95% coverage of the human vaginal microbiome and be applicable to populations from North America, Africa, and Asia. The methods and systems of the present disclosure can thus provide a comprehensive reference repository and a convenient cataloging tool for fast and accurate characterization of vaginal metagenomes and metatranscriptomes. The reference gene catalogs produced according to the present disclosure can be compilations of vaginal bacterial species pangenomes, creating a vaginal "meta-pan-genome." Methods and systems of the present disclosure can be further used to characterize the amount of intraspecies diversity present in individual vaginal communities; whereas previous characterization of these communities using either 16S rRNA gene taxonomic profiling or assembly-based metagenomic analyses has failed to resolve this diversity, the present inventors have shown that vaginal communities contain far more intraspecies diversity than originally expected. This insight challenges the conventional idea that the vaginal microbiota are dominated by one strain, or even one species, of *Lactobacillus,* and has major implications for the ecology of these otherwise low-diversity bacterial communities. Ultimately, reference gene catalogs produced according to the present disclosure and their associated analytical frameworks can facilitate and standardize the analysis and interpretation of large metagenomic and metatranscriptomic datasets, thus expanding understanding of the role of vaginal microbial communities in health and disease.

Methods for constructing or generating a reference gene catalog according to the present disclosure generally comprise at least one data collection step, at least one data processing step, and at least one redundancy removal step. **In** the at least one data collection step, metagenomes and/or isolate genomes from one or more microbial communities are generally obtained and/or newly sequenced. The at least one data processing step generally comprises at least one step or sub-step selected from the following: (i) preprocessing sequence data for each of one or more samples, e.g. by removing human contaminants from a sample, quality-filtering, and/or removing ribosomal RNA; (ii) assembling at least one metagenome from each of one or more samples, e.g. by executing a procedure to generate one or more nucleotide based de novo assemblies; (iii) compiling coding DNA sequences (CDSs); (iv) taxonomic annotation, e.g. by transitive assignment of species name from reads mapping to contigs; and (v) functional annotation, e.g. using annotations from one or more functional databases. The at least one redundancy removal step generally comprises at least one of clustering highly similar genes to avoid spurious inflation and keeping the longest gene of a gene cluster to remove gene fragments. The method may further comprise at least one orthologous protein family grouping step, which generally comprises clustering genes using Jaccard index coefficiency. Reference gene catalogs generated according to the present disclosure generally include, for each orthologous protein family group, at least one type of information selected from the following: (i) general attributes (e.g. gene symbol, taxonomic group, gene richness category); (ii) pathway attributes (e.g. ID and annotation); (iii) protein attributes (e.g. category and annotation); (iv) orthologous group attributes (e.g. protein family size, alignment score, taxonomic information, functional category); (v) alignment(s); (vi) phylogeny or phylogenies; (vii) nucleotide sequence(s); and (ix) amino acid sequence(s).

Also disclosed herein but not claimed are methods and systems for treating a human patient with a live biotherapeutic formulation. In general, such methods include constructing a reference gene catalog by the methods described herein, comparing a gene-specific characterization of a microbiome of the patient with the reference gene catalog, identifying a deficiency or overabundance of a bacterial strain in the patient's microbial community relative to the reference gene catalog, formulating a remedial live biotherapeutic formulation comprising bacteria selected to address the deficiency or overabundance, and administering the remedial live biotherapeutic to the patient.

The methods and systems of the present disclosure allow users not only to obtain greater insight into human-associated microbial communities, but to translate these insights into clinical biomarkers and treatments, e.g. live biotherapeutics. Deeper understandings of the complex mechanisms of host-microbiota interactions require the integration of multi-omics data. The present disclosure provides for the generation of reference gene catalogs that serve as a central reference database and an analytical framework to enable the efficient and accurate characterization of the microbial gene content of a microbiome, e.g. the human vaginal microbiome, and allows for the integrated analysis of metagenomic and metatranscriptomic data. The present disclosure further provides a gene-specific approach to describe the structure of microbial communities, e.g. vaginal microbial communities, with fine-scale variation at the intraspecies level. Such insights into intraspecies diversity within a microbial community are far beyond the capabilities of current genome references and investigation tools. The present disclosure also facilitates the analysis of multi-omics data now common to microbiome studies; provides comprehensive insight into community membership, function, and ecological perspective of a microbiome, e.g. the vaginal microbiome; and is useful to formulate gene-specific, and therefore more targeted and effective, live biotherapeutics.

Microbiome studies have become increasingly sophisticated with the rapid advancement of sequencing throughput and the associated decrease in sequencing cost. However, identifying features that drive correlations between the microbiome and health using multi-omics sequencing data remains challenging. This is due, in part, to difficulties in analyzing and integrating the complex metagenomic and metatranscriptomic data now common to microbiome studies. A scalable tool that provides a comprehensive characterization of such multi-omics data is therefore highly desired. Reference gene catalogs generated according to the present disclosure can be large microbiome databases designed to fulfill such research needs for investigations of microbiomes and their relation to health, e.g. the vaginal microbiome and its relation to reproductive and urogenital health in women. In summary, reference gene catalogs generated according to the present disclosure may provide any one or more of the following advantages and benefits relative to the solutions of the prior art: (i) comprehensive breadth, including previously observed community types, species, and even fungi and viruses; (ii) a gene-specific design that enables the integration of functional and taxonomic characterization of the metagenomic and metatranscriptomic data originating from the same sample; (iii) a high scalability and low memory requirement; (iv) a high sensitivity that affords characterization of the gene content of low-abundance bacteria; (v) an easy-to-use framework from which to evaluate gene richness and within-species diversity.

Reference gene catalogs generated according to the present disclosure can contain a multitude of non-redundant genes that can be identified from metagenomes and bacterial isolates. These non-redundant genes can also be clustered into orthologous groups, e.g. vaginal orthologous groups (VOGs) when the microbiome being investigated is the vaginal microbiome, using a memory-efficient network-based algorithm that handles node connectivity in high-dimensionality space. This approach to identifying orthologous protein sequences allows for great flexibility because it does not rely on a single sequence similarity cutoff value. These families of orthologs can assist the development of a mechanistic understanding of these proteins and how they relate to health. For example, the *L. crispatus* pullulanase (*pulA*) has recently been identified, characterized, and shown to encode an enzyme with amylase activity, which likely allows the species to degrade host glycogen in the vaginal environment. Using the methods of the present disclosure, the present inventors have been able to identify pullulanase domain-containing proteins in 37 other vaginal taxa, including *G*. *vaginalis, L. iners,* and *P. timonensis,* providing insight into the breadth of vaginal bacteria that may be capable of degrading host glycogen. In this way, the methods and systems of the present disclosure can facilitate knowledge retrieval, hypothesis generation, future experimental validation, and the development of novel and/or tailored gene-specific live biotherapeutics for administration to a patient in need thereof.

Using the methods and systems of the present disclosure, the intraspecies diversity present within individual microbial communities, e.g. individual vaginal microbial communities, can be identified and characterized. Populations of bacterial species in, for example, vaginal communities likely comprise multiple strains. Previous studies of the vaginal microbiome have largely treated these species as singular genotypes, although some more recent studies have examined intraspecies diversity in these communities. Intraspecies diversity is important because it is likely to influence many properties of the communities, including their temporal stability and resilience and their relationship to host health. However, intraspecies diversity is difficult to detect using typical assembly-based metagenomic analysis strategies, which are notoriously ill-suited for resolving strains of the same species. The methods and systems of the present disclosure can be a more suitable tool for characterizing intraspecies diversity because they are built to contain the non-redundant pangenomes of most species common to the microbial environment, e.g. the vagina. Strict mapping of sequence reads against reference gene catalogs generated according to the present disclosure provides an accurate and sensitive way of identifying the aggregated non-redundant genes that belong to each species in a metagenome, and it is expressly contemplated that the methods and systems of the present disclosure may enable future investigations of intraspecies diversity, and leveraging of such intraspecies diversity (e.g. by formulating live biotherapeutics to regulate and/or maintain a degree of intraspecies diversity associated with health), in microbial communities including but not limited to the vaginal microbial community.

Methods and systems of the present disclosure can be used to determine not only the identity and characteristics of intraspecies diversity, but also the structure thereof. As described in the non-limiting Examples that follow, vaginal metagenomes from different subjects contain related sets of species-specific non-redundant genes. Without wishing to be bound by any particular theory, it is believed that these clusters of samples with shared gene content represent similar collectives of strains, which the present inventors term "metagenomic subspecies," and it is expected that, given their shared gene content, these metagenomic subspecies might also share phenotypic characteristics. As a result, live biotherapeutics can be formulated that include, or exclude, one or more identified metagenomic subspecies to provide, or avoid, an identified effect of the metagenomic subspecies on the health of the microbial environment, e.g. the vaginal microbial environment.

One advantage and benefit of the present disclosure is its usefulness to generate reference gene catalogs that are both central repositories and highly scalable tools for fast, accurate characterization of a microbiome, e.g. a vaginal microbiome. The methods and systems of the present disclosure may be particularly useful for users with limited computational skills, a large volume of sequencing data, and/or limited computing infrastructure. In particular, the metagenome-metatranscriptome data integration enabled by the gene-specific design of the methods and systems of the disclosure provides a powerful approach to determine the expression patterns of microbial functions, and in doing so to characterize contextualized complex mechanisms of host-microbiota interactions in microbial communities, e.g. vaginal communities. This feature makes possible the meta-analysis of microbiome features and the quantitative integration of findings from multiple studies, which helps alleviate the common issue of confounding gene copy number that has been a major challenge in analyzing metatranscriptomic datasets to date. It is also anticipated that the methods and systems of the present disclosure may be used to process metaproteomic datasets when that practice becomes common and easily accessible. Each of the protein sequences of each gene could be used to map peptides obtained from metaproteomic pipelines. It is also expressly contemplated that the methods and systems of the present disclosure may be useful to identify nucleotide variants within a gene-which will further facilitate understanding of within-species diversity and change in a microbial ecosystem, e.g. a vaginal ecosystem, and enable even more selective and/or targeted formulation of live biotherapeutics-and embodiments incorporating this capability are within the scope of the present disclosure. Furthermore, the methods and systems of the present disclosure are useful to generate reference gene catalogs including gene sequences of non-bacterial microbes, e.g. viral and fungal gene sequences, providing a more complete understanding of the microbial community of interest.

### Formulation of Live Biotherapeutics for Vaginal Microbiome Treatment

In reproductive-age women, *Lactobacillus* spp. are characteristic of an optimal vaginal microbiota. *Lactobacillus* spp. produce bacteriocins to suppress pathogenic growth of certain bacteria, as well as lactic acid. Lactic acid lowers the vaginal pH to around 4.5 or less, hampering the survival of other bacteria.

The vaginal microbiome differs in important ways from other microbiomes; for example, while an optimal gut microbiome is a highly diverse, high-biomass microbial community, an optimal vaginal microbiome is characterized by low bacterial diversity often dominated by one species of *Lactobacillus.* Specifically, previous metataxonomic studies utilizing 16S rRNA gene sequencing analysis have revealed that there are five major Community State Types (CSTs) of the vaginal microbiome, of which four are dominated by one species of *Lactobacillus:* CST I (dominated by *L. crispatus*)*,* CST II (dominated by *L. gasseri*)*,* CST III (dominated by *L. iners*)*,* and CST V (dominated by *L. jensenii*)*.* CST IV, however, which includes the vaginal microbiomes of about 25% of women, is characterized by a relative dearth of *Lactobacillus* spp. Low abundance of *Lactobacillus* in the vaginal microbiome is associated with increased risk for severe adverse gynecologic and obstetric outcomes. Adverse gynecologic outcomes associated with low *Lactobacillus* abundance include, but are not limited to, acquisition of sexually transmitted infections (STIs) (including human immunodeficiency virus (HIV), chlamydia, gonorrhea, herpes simplex virus (HSV), and human papillomavirus (HPV)), bacterial vaginosis (the most frequently cited cause of vaginal discharge and malodor), yeast infection, urinary tract infection (UTI), and pelvic inflammatory disease (PID). Adverse obstetric outcomes associated with low *Lactobacillus* abundance include, but are not limited to, preterm delivery and low birth weight, infertility, stillbirth, premature rupture of membranes (PROM), postpartum and postabortal endometritis, amniotic fluid infection, and chorioamnionitis. *Lactobacillus* spp. are thus key to reproductive and gynecological health, and not all CSTs are equally protective; CST IV is associated with high risk to these and other adverse health outcomes, and CST III is suboptimal compared to CSTs I, II, and V (see, e.g., Vonetta L. Edwards et al., "The cervicovaginal microbiota-host interaction modulates *Chlamydia trachomatis* infection," 10(4) *mBio* e01548-19 (Aug. 2019), and Kenetta L. Nunn et al., "Enhanced trapping of HIV-1 by human cervicovaginal mucus is associated with *Lactobacillus crispatus-dominant* microbiota," 6(5) *mBio* e01084-15 (Oct. 2015)). Moreover, it is known that the distribution of vaginal microbiome CSTs varies with race; for example, 40.5% of black women and 38.1 % of Hispanic women harbor a CST IV vaginal microbiome, compared to 19.8% of Asian women and 10.3% of white women.

While several proposed solutions to the restoration and maintenance of vaginal microbiota associated with positive health outcomes exist, these proposed solutions suffer from several drawbacks. The selection of strains used in the formulation of live biotherapeutics (LBPs) is empiric and is based on criteria such as adhesion or antimicrobial production, but little or no information is available on the women and the microbiota from which the strains were cultured. Often, the LBPs comprise strains of a particular species (e.g. *L. crispatus, L. gasseri,* or *L. jensenii*) that are not typically found in the vaginal microbiome. In these approaches, there is no ecological or scientific rationale for strain selection, and the efficacy of LBPs formulated according to these approaches has yet to be demonstrated as superior to current drug treatments.

A list of selected currently available LBPs for maintenance of the vaginal microbiome is given in Table 1.

**Table 1**

| **Formulation name** | **Ingredients** |
|---|---|
| PHYSIOFLOR | *L. crispatus* IP 174178 |
| KRAMEGIN | *L. acidophilus* + Krameria triandra plant extract + lactic acid |
| GYNOFLOR | *L. acidophilus* KS400 + estriol |
| LACTAGYN | *L. acidophilus, L. rhamnosus, S. thermophilus, L. delbrueckii* subsp. Bulgaricus |
| GYNOPHILUS | *L. casei rhamnosus* Lcr35 |
| ACTICAND | *L. fermentum* LF10 + *L. acidophilus* LA02 |
| ESTROMINERAL PROBIOGEL | *L. fermentum* LF10 *+ L. plantarum* LP02 |
| ECOVAG | *L. gasseri* EB01-DSM 14869 + *L. rhamnosus* Lbp PB01-DSM 14870 |
| GYNO-CANESFLOR | *L. plantarum* P17630 |
| FEMILAC | *L. rhamnosus + L. delbrueckii + L. acidophilus + S. thermophilus* |
| SYNBIO GIN | *L. rhamnosus* IMC 501 *+ L. paracasei* IMC 502 |
| GYNOPHILUS LP | *L. rhamnosus* Lcr35 regenerans |
| ECOLOGIC FEMI+ | *B. bifidum* W28 + *L. acidophilus* W70 + *L. helveticus* W74 + *L. brevis* W63 |
| FLORISIA | *L. brevis* CD2 + *L. salivarius* subsp. salicinius FV2 + *L. plantarum* FV9 |
| LACTIN V | *L. crispatus* CTV-05 |
| RC-14/GR-1 | *L. fermentum* RC-14 + *L. rhamnosus* GR-1 |
| DAYE PROBIOTIC | *L. plantarum* GLP3 |

Even CST alone, while more informative than its gut microbiome equivalent, lacks the functional information necessary to formulate an effective LBP, because the particular strain of *Lactobacillus* spp. is the driver of functional specificity and certain strains are better than others.

Reference gene catalogs of genes in the vaginal microbiome, generated according to the methods and systems of the present disclosure, overcome these drawbacks and allow for the formulation of much more effective LBPs. Particularly, because the reference gene catalogs of the present disclosure are comprehensive, have broad application to different populations and ethnicities, and reveal extensive within-woman intraspecies diversity, those who wish to formulate LBPs for restoration of the vaginal microbiota can identify multiple strains of a species that may contribute to (or detract from) the health of the vaginal environment and provide functional redundancy that guarantees stability of the species *in situ* in the vaginal environment. The present disclosure allows reference gene catalogs created thereby to be leveraged to rationally design and select one or more bacterial strains, or a mixture ("consortium") thereof, as therapeutics.

The present inventors have identified certain features that may be desired in LBP formulations to provide stability and resilience to the vaginal microbiome. By way of non-limiting example, not all *L. crispatus* strains are equally beneficial to host health; vaginal microbiota dominated by *L. crispatus* can be highly stable, but can also lack resilience upon disturbance, such as the use of a lubricant or sexual intercourse. It is desirable to provide vaginal microbiota dominated by stable and resilient *L. crispatus,* but prior to the present invention, no known characteristics could predict the stability of *L. crispatus.*

### Vaginal Infections, Disease, and the Microbiota

Using microscopic observation, the composition of the vaginal microbiota has long been linked to disease risk, with the presence of *Lactobacillus* spp. providing protection while a paucity of *Lactobacillus* spp. and the presence of a diverse set of Gram-negative anaerobic species are associated with increased risk of urogenital disease generally and bacterial vaginosis, a condition present in over 70% of women attending sexually transmitted infection clinics, over 50% of African-American women, and 29% of all women aged 14 to 49 years in the general U.S. population. High-throughput molecular analyses, and in particular the integrated analysis of metagenomic and metatranscriptomic data according to and made possibly by the systems and methods of the disclosure, afford a more in-depth and precise characterization of the vaginal microbiota and insight into the role of specific clades, species, strains, or genes in disease risk. These high-resolution analyses advance our understanding of risks for various diseases, some examples of which are now described in further detail.

### Bacterial Vaginosis

Diagnosis of bacterial vaginosis (BV) relies on the Amsel criteria in the clinical setting and on the Nugent scoring system in research settings. Interestingly, despite the use of molecular analysis to define BV states, no one taxon has been confirmed as the etiological agent of the condition, and BV remains ill-defined microbiologically as a polymicrobial state, basically characterized by the lack of predominant *Lactobacillus* spp. That said, several bacteria, such as *Gardnerella vaginalis,* have been shown to be associated with the condition in some studies but not others. Early studies failed to reproduce the disease after direct vaginal inoculation of *G. vaginalis* isolated from women with BV, while inoculation with whole vaginal secretions did reproduce the disease, supporting that the condition is either polymicrobial or other factors contribute. It is highly likely that the pathogenic potential of *G. vaginalis* might differ depending on the specific strain of *G. vaginalis* and possibly the vaginal immune state or ethnicity of the subject. Interestingly, it appears that *G. vaginalis* can be transmitted sexually. A longitudinal study of young women in Australia found that *Gardnerella* was more likely to be found in those having penile sex. *Gardnerella* was also found in approximately a third of girls aged 10 to 12 years in a pre-menarche vaginal microbiota study, indicating that this organism can be acquired and/or facilitated by means other than sexual activity, potentially by transfer at birth from mother to daughter. Yet *G. vaginalis* colonization increased after sexual activity in a cohort of young women who were monitored pre- and post-sexual debut, indicating sexual activity is a factor in the transmission of this bacterium. In support of the polymicrobial nature of BV, in a longitudinal study of women who have sex with women, BV incidence was associated with sexual behaviors, and most strongly correlated with a new sexual partner with BV-associated symptoms. Furthermore, a large cohort study of 1,093 women in general practice care in Australia found that either a recent new female sexual partner or multiple male partners were significantly associated with prevalent or incident BV cases. Interestingly, in this study estrogen contraceptives were protective. A similar study in the United States found that new sexual partners and oral vulvovaginal sex were both significant risk factors for BV, while an *L. crispatus-dominated* (CST I) microbiota was protective. The lack of a clear definition for BV makes studying its etiology challenging. One study attempted to improve the definition of BV using molecular methods, combining bacterial composition (16S rRNA gene amplicon sequencing), eukaryotic composition (ITS sequencing), and *Trichomonas* characterization (sequencing of the *tvk* loci), but it failed because of limitations associated with the study such as the lack of speciation of *Lactobacillus* spp., highlighting the need for further development of species-specific characterizations, and even more advantageously the strain- and gene-specific characterizations enabled by the systems and methods of the present disclosure. An improved definition of BV would have major implications in women's clinical management and women's health as a whole.

While new antibiotics are being developed or tested to treat BV, the present invention enables the leveraging of vaginal microbiota for the development of live biotherapeutic formulations to moderate the microbiota to restore a *Lactobacillus-*dominated protective vaginal microbiota, and even more specifically, in particular embodiments, a vaginal microbiota characterized by *L. crispatus* strains as described and disclosed herein. As drug-based treatment failure can be high, with antibiotic resistance appearing and recurrence very common, the alternative, live biotherapeutic-based approaches enabled by the present invention are highly advantageous and beneficial. Vaginally delivered live biotherapeutics have a favorable safety profile and can be used in combination therapy after antibiotic treatment, but prior live biotherapeutic formulations have met with limited success in preventing or treating BV, certainly because these formulations do not take into account the ecology of the vaginal microbiota and often rely on non-vaginal *Lactobacillus* strains or on too few, or ineffective, strains of *L. crispatus.* The present invention describes, and allows for the further development and optimization of, efficacious live biotherapeutic formulations for the prevention and/or treatment of bacterial vaginosis.

### Pelvic Inflammatory Disease

The composition of the vaginal microbiota appears to play a role in the development of another important disease, pelvic inflammatory disease (PID). PID is associated with inflammation in the upper reproductive tract in women, characterized by sudden onset of pain along with cervical, adnexal, or uterine tenderness. Risk factors for PID include those that also affect the composition of the vaginal microbiota, such as history of multiple sexual partners or early age of commencement of sexual activity. Microbial risk factors for PID include sexually transmitted infections and bacterial vaginosis. In addition to the endometrial presence of sexually transmitted pathogens such as *C. trachomatis, Mycoplasma genitalium,* and *Neisseria gonorrhoeae,* polymerase chain reaction (PCR) testing for BV-associated bacteria in endometrial samples identified bacteria such as *Sneathia sanguinegens, Sneathia amnionii, Atopobium vaginae,* and BV-associated bacteria 1 (BVAB1) in women with PID. It is common to fail to identify known pathogens in women with PID, although frequently many other organisms are detected in the upper reproductive tract. Hence, it is likely that a *Lactobacillus-dominated* vaginal microbiota, and even more specifically, embodiments, a vaginal microbiota characterized by *L. crispatus* strains as described and disclosed herein, could be protective for PID.

### Sexually Transmitted Infections

Risk for contraction of sexually transmitted pathogens has been associated with the composition of the vaginal microbiota. The most insights have come from studies into incidence and prevalence of *C. trachomatis. Chlamydia* is one of the most common sexually transmitted bacterial pathogens and has been found in three independent studies to be more likely detected in association with *L. iners* (CST III) and/or CST IV. This may relate to availability of metabolites produced by these types of microbiota that benefit the pathogen, as found in one study. On the other hand, the vaginal microbiota, in particular *L. crispatus* (CST I), may have specific anti-chlamydial, anti-gonococcal, and immune enhancing properties, as evidenced *in vitro* and on a porcine epithelial model. *N. gonorrhoeae* infections are less common (compared to *Chlamydia)* in women. While there is relatively little information on the composition of the vaginal microbiota in the context of gonococcal infections, it has been shown *in vitro* that *Lactobacillus* spp. (especially *L. gasseri* (CST II) and *L. jensenii* (CST V)) can directly compete *with N. gonorrhoeae* for epithelial binding. A large nested case-control study in African women identified that there are several taxa within the vaginal microbiota that are associated with increased risk of HIV acquisition. Bacterial vaginosis, sexually transmitted infections such as *Chlamydia* and herpes simplex, and vaginal washing have also been associated with increased risk of transmission and/or acquisition of HIV. Altogether, these data support mechanisms such as competition, low pH, and specific anti-bacterial molecules (e.g. bacteriocin), through which the vaginal microbiota is a major driver of protection against infectious agents.

### Pregnancy Outcomes and the Cervicovaginal Microbiota

In pregnancy, the lack of menses and the increase in circulating estrogen are associated with a microbiota characterized by an increased dominance of *Lactobacillus* spp. as gestation progresses. This is a feature of the vaginal microbiota in pregnancy that has been established by several studies of varied power and sampling intensities, both in the United States and in Europe. Interestingly, this inherent stability of the microbiota in pregnancy was also true at other body sites. Post-partum, and up to a year after delivery, the vaginal microbiota was characterized by a paucity of *Lactobacillus* spp. (CST IV), even in pregnancies where *Lactobacillus* spp. were dominant during gestation. These findings support the hypothesis that adverse postpartum outcomes such as endometritis and sepsis might be mediated by vaginal microbes, and suggest that the vaginal microbiota, and particularly the *Lactobacillus* spp. composition thereof, plays an important role in biological and reproductive outcomes as well; it is one advantage of the present disclosure to enable metagenomic and metatranscriptomic analysis of the vaginal microbiome to identify gene- and/or strain-specific patterns in the vaginal microbiota associated with particular biological and reproductive outcomes and allow for the formulation of live biotherapeutic formulations to remediate a microbiome characterized by a pattern associated with negative outcomes and/or provide for a microbiome characterized by a pattern associated with positive outcomes. Generally, *Lactobacillus* spp. abundance increases, and community diversity decreases, during pregnancy, with a postpartum shift to high diversity.

Several studies have documented the composition of the vaginal microbiota associated with adverse pregnancy outcomes such as preterm birth. While certain studies found association between a few bacteria, mostly anaerobes, and adverse pregnancy outcomes, others did not; it is one advantage of the present disclosure to enable characterization of the vaginal microbiota at a gene- and/or strain-specific level and thereby to identify particular microbial genes or strains associated with particular pregnancy outcomes, and to formulate live biotherapeutic formulations accordingly. In this way, the present disclosure improves the ability to distinguish cause from effect, which prior solutions in the art have found challenging, likely because such solutions suffer from a low number and poor phenotype (i.e. not all preterm births were spontaneous) of preterm birth cases.

Preterm premature rupture of membranes (PPROM) is one adverse pregnancy outcome that has strongly correlated with the cervicovaginal microbiome in distinct studies. In one study, and consistent with several others, women who experienced PPROM were less likely to have *Lactobacillus,* and more likely to have lower abundance of *Lactobacillus* and high diversity of the microbiota, in the vaginal microbiome composition. Furthermore, the presence of *Mollicutes* such as *Mycoplasma* or *Ureaplasma,* although common in healthy vaginal microbiomes, have been found to be more frequently present in the vaginal microbiome of women who experienced PPROM. Both early and late miscarriages have long been associated with BV or the presence of specific flora in the vagina. Chorioamnionitis or intra-amniotic infections have also been associated with the vaginal microbial community state, including a recent history of BV. The present disclosure enables well-powered study designs, the inclusion of ethnically diverse populations, the identification of predictive gene- and/or strain-specific signatures in the cervicovaginal microbiota or its products, and thus ultimately the provision of novel live biotherapeutic formulations to restore a protective vaginal microbiota

### Cervicovaginal Microbiota in Infertility and Fertility Treatment

The composition of the vaginal microbiota is thought to influence fertility and outcomes of fertility treatment. Most published studies were performed in the context of *in vitro* fertilization (IVF) procedures, which could be well-controlled. In these studies, BV-associated bacteria in the vagina have been shown to be associated with a reduced pregnancy rate. Haahr focused on using Nugent score and PCR detection of BV-associated bacteria in the vagina and comparing to IVF success in 130 women, and found that Nugent and PCR correlated highly and that women with PCR-detected BV-associated bacteria were significantly less likely (9% compared to an overall rate of 35%) to obtain a clinical pregnancy. Interestingly, women undergoing IVF with tubal factor infertility-a pathology associated with infections-were found to be more likely to have a vaginal microbiota consistent with BV by analysis of smears, supporting a connection between these etiologies. This finding supports that precision medicine approaches around fertility treatment and the vaginal microbiota can inform practice in the fertility clinic. Further to this finding, using next-generation sequencing of the vaginal microbiota, women with idiopathic infertility were found to be more likely to have a microbiota profile consistent with BV compared to healthy women. Another study identified trends for distinct microbiota in women with a history of infertility compared to women with a history of fertility, albeit a retrospective study on a small sample size. A study of the composition of the vaginal microbiota on the day of embryo transfer in women undergoing IVF found that a lower vaginal microbiota diversity index correlated with a resultant live birth; although this study did not profile the taxa present but merely compared the diversity index, providing a relatively limited insight into the role of vaginal microbiota in reproductive outcomes, the present disclosure enables the refinement of these findings by profiling the taxa present at a gene- and/or strain-specific level, thus in turn enabling the identification of specific genes and/or strains associated with particular reproductive outcomes and the formulation of live biotherapeutics configured to decrease the likelihood of a negative outcome and/or increase the likelihood of a positive outcome. The present disclosure also enables gene- and/or strain-specific analysis of the upper reproductive tract microbiota to shed further insights into fertility and fertility treatment outcomes and the formulation of live biotherapeutics for administration to the upper reproductive tract. The insights and methods for formulating live biotherapeutics provided by the present disclosure are not necessarily limited to the controlled context of IVF procedures, but can take into account certain other parameters (e.g. time to pregnancy) that enable the development of live biotherapeutics that can improve natural conception outcomes.

### Cervical Intraepithelial Neoplasia, Cervical Cancer, and Other Genitourinary Cancers

A marked decrease of *Lactobacillus crispatus* has been found in the vaginal microbiomes of women with cervical intraepithelial neoplasia (CIN) and cervical cancer compared with the vaginal microbiomes of healthy women. In addition, the diversity of microorganisms in the vaginal microbiota is increased in women with CIN and/or cervical cancer, and in particular *Atopobium vaginae, Dialister invisus, Finegoldia magna, Gardnerella vaginalis, Prevotella buccalis,* and *Prevotella timonensis* were significantly associated with the risk of CIN and cervical cancer. Other anaerobic bacteria, such as *Peptostreptococcus anaerobius* and *Porphyromonas uenonis,* are also observed to be more abundant in the vaginal microbiota of women with CIN or cervical cancer.

### Improvements in Health and Reproductive Outcomes Achievable by the Invention

The present invention provides live biotherapeutic formulations for achieving or increasing the likelihood of a desired health or reproductive outcome in a female human subject and/or for ameliorating, preventing, treating, or decreasing the likelihood of an undesired health or reproductive outcome in a female human subject. The disclosure further provides methods for creating the live biotherapeutic formulation, which may comprise any one or more of the steps of (a) generating a gene-specific characterization, at an intraspecies level, of the subject's vaginal microbial community; (b) comparing the gene-specific characterization to a reference gene catalog of the human vaginal microbiome, wherein the reference gene catalog comprises at least one metagenome or single-strain genome associated with a healthy microbiome; (c) identifying, based on the comparison, a deficiency or excess of at least one bacterial strain in the subject's vaginal microbial community; and (d) formulating a remedial live biotherapeutic formulation, comprising bacteria adapted to remedy the deficiency or excess of the at least one bacterial strain the subject's vaginal microbial community. The disclosure still further provides methods for achieving or increasing the likelihood of a desired health or reproductive outcome in the subject and/or for ameliorating, preventing, treating, or decreasing the likelihood of an undesired health or reproductive outcome in the subject, which may comprise, instead of or in addition to any one or more of the steps (a) through (d) listed above, administering the live biotherapeutic formulation to the subject.

Some non-limiting examples of undesired health or reproductive outcomes in female human subjects that may be ameliorated, prevented, or treated, or whose likelihood may be decreased, by the live biotherapeutic formulations and methods of manufacture and use thereof provided by the present invention are as follows:
(1) Bacterial vaginosis. Bacteria in the live biotherapeutic formulation used to ameliorate, prevent, treat, or decrease the likelihood of this outcome may comprise a selected strain or consortium of strains comprising at least one of (i) *Lactobacillus crispatus* bacteria configured to express at least one of the asparagine synthase B (*asnB*) gene of SEQ ID NO: 2 and the asparagine synthase B (*asnB*) gene of SEQ ID NO: 3; (ii) *Lactobacillus crispatus* bacteria containing an asparagine synthase B (*asnB*) gene encoding at least one of a polypeptide of SEQ ID NO: 5 and a polypeptide of SEQ ID NO: 6; and (iii) at least one strain selected from the group consisting of LUCA 111, LUCA011, LUCA015, LUCA009, LUCA102, LUCA006, LUCA059, LUCA103, and LUCA008. Without wishing to be bound by any particular theory, it is believed that such bacteria can ameliorate, prevent, treat, or decrease the likelihood of this outcome by any one or more of (i) remediating a lack of predominant *Lactobacillus* spp., (ii) providing an *L. crispatus-dominated* (CST I) microbiota; and/or (iii) inhibiting, limiting, or preventing the growth of *Gardnerella vaginalis,* e.g. by affecting glutamine binding and thus production of asparagine by the ammonia sequestration mechanism.
(2) Pelvic inflammatory disease. Bacteria in the live biotherapeutic formulation used to ameliorate, prevent, treat, or decrease the likelihood of this outcome may comprise a selected strain or consortium of strains comprising at least one of (i) *Lactobacillus crispatus* bacteria configured to express at least one of the asparagine synthase B (*asnB*) gene of SEQ ID NO: 2 and the asparagine synthase B (*asnB*) gene of SEQ ID NO: 3; (ii) *Lactobacillus crispatus* bacteria containing an asparagine synthase B (*asnB*) gene encoding at least one of a polypeptide of SEQ ID NO: 5 and a polypeptide of SEQ ID NO: 6; and (iii) at least one strain selected from the group consisting of LUCA 111, LUCA011, LUCA015, LUCA009, LUCA102, LUCA006, LUCA059, LUCA103, and LUCA008. Without wishing to be bound by any particular theory, it is believed that such bacteria can ameliorate, prevent, treat, or decrease the likelihood of this outcome by any one or more of (i) remediating a lack of predominant *Lactobacillus* spp., (ii) providing a *Lactobacillus-dominated* microbiota; and (iii) inhibiting, limiting, or preventing the growth of any one or more of *Chlamydia trachomatis, Mycoplasma genitalium, Neisseria gonorrhoeae, Sneathia sanguinegens, Sneathia amnionii, Atopobium vaginae,* and BVAB1, e.g. by affecting glutamine binding and thus production of asparagine by the ammonia sequestration mechanism.
(3) Sexually transmitted infection. Bacteria in the live biotherapeutic formulation used to ameliorate, prevent, treat, or decrease the likelihood of this outcome may comprise a selected strain or consortium of strains comprising at least one of (i) *Lactobacillus crispatus* bacteria configured to express at least one of the asparagine synthase B (*asnB*) gene of SEQ ID NO: 2 and the asparagine synthase B (*asnB*) gene of SEQ ID NO: 3; (ii) *Lactobacillus crispatus* bacteria containing an asparagine synthase B (*asnB*) gene encoding at least one of a polypeptide of SEQ ID NO: 5 and a polypeptide of SEQ ID NO: 6; and (iii) at least one strain selected from the group consisting of LUCA 111, LUCA011, LUCA015, LUCA009, LUCA102, LUCA006, LUCA059, LUCA103, and LUCA008. Without wishing to be bound by any particular theory, it is believed that such bacteria can ameliorate, prevent, treat, or decrease the likelihood of this outcome by any one or more of (i) remediating an *L.* iners-dominated (CST III) or *Lactobacillus-poor* (CST IV) microbiota, e.g. thereby reducing the availability of metabolites produced by *L. iners* or other bacteria that benefit *C. trachomatis* or other sexually transmitted pathogen; (ii) providing an *L. crispatus-dominated* (CST I) microbiota that provides an anti-chlamydial, anti-gonococcal, and/or immune-enhancing effect; (iii) providing an *L. gasseri-dominated* (CST II) or *L. jensenii-*dominated (CST V) microbiota, e.g. wherein *L. gasseri* and/or *L. jensenii* compete with *N. gonorrhoeae* for epithelial binding; (iv) competing for a resource, e.g. ammonia, in the vaginal environment with a sexually transmitted pathogen; (v) reducing the pH of the vaginal environment to inhibit, limit, or prevent growth of a sexually transmitted pathogen; and (vi) producing an antibacterial molecule (e.g. bacteriocin) that inhibits, limits, or prevents growth of a sexually transmitted pathogen.
(4) Postpartum endometritis and/or sepsis. Bacteria in the live biotherapeutic formulation used to ameliorate, prevent, treat, or decrease the likelihood of this outcome may comprise a selected strain or consortium of strains comprising at least one of (i) *Lactobacillus crispatus* bacteria configured to express at least one of the asparagine synthase B (*asnB*) gene of SEQ ID NO: 2 and the asparagine synthase B (*asnB*) gene of SEQ ID NO: 3; (ii) *Lactobacillus crispatus* bacteria containing an asparagine synthase B (*asnB*) gene encoding at least one of a polypeptide of SEQ ID NO: 5 and a polypeptide of SEQ ID NO: 6; and (iii) at least one strain selected from the group consisting of LUCA 111, LUCA011, LUCA015, LUCA009, LUCA102, LUCA006, LUCA059, LUCA103, and LUCA008. Without wishing to be bound by any particular theory, it is believed that such bacteria can ameliorate, prevent, treat, or decrease the likelihood of this outcome by any one or more of (i) decreasing microbial diversity in the postpartum vaginal microbiota; (ii) increasing the abundance, predominance, and/or proportion of *Lactobacillus* in the postpartum vaginal microbiota; and (iii) remediating a *Lactobacillus-*poor (CST IV) microbiota.
(5) Preterm birth. Bacteria in the live biotherapeutic formulation used to ameliorate, prevent, treat, or decrease the likelihood of this outcome may comprise a selected strain or consortium of strains comprising at least one of (i) *Lactobacillus crispatus* bacteria configured to express at least one of the asparagine synthase B (*asnB*) gene of SEQ ID NO: 2 and the asparagine synthase B (*asnB*) gene of SEQ ID NO: 3; (ii) *Lactobacillus crispatus* bacteria containing an asparagine synthase B (*asnB*) gene encoding at least one of a polypeptide of SEQ ID NO: 5 and a polypeptide of SEQ ID NO: 6; and (iii) at least one strain selected from the group consisting of LUCA 111, LUCA011, LUCA015, LUCA009, LUCA102, LUCA006, LUCA059, LUCA103, and LUCA008. Without wishing to be bound by any particular theory, it is believed that such bacteria can ameliorate, prevent, treat, or decrease the likelihood of this outcome by any one or more of (i) remediating a *Lactobacillus-poor* (CST IV) microbiota; and (ii) inhibiting, limiting, or preventing the growth of, or otherwise decreasing or preventing an increase in the abundance of, *Gardnerella* and/or *Ureaplasma.*
(6) Preterm premature rupture of membranes. Bacteria in the live biotherapeutic formulation used to ameliorate, prevent, treat, or decrease the likelihood of this outcome may comprise a selected strain or consortium of strains comprising at least one of (i) *Lactobacillus crispatus* bacteria configured to express at least one of the asparagine synthase B (*asnB*) gene of SEQ ID NO: 2 and the asparagine synthase B (*asnB*) gene of SEQ ID NO: 3; (ii) *Lactobacillus crispatus* bacteria containing an asparagine synthase B (*asnB*) gene encoding at least one of a polypeptide of SEQ ID NO: 5 and a polypeptide of SEQ ID NO: 6; and (iii) at least one strain selected from the group consisting of LUCA 111, LUCA011, LUCA015, LUCA009, LUCA102, LUCA006, LUCA059, LUCA103, and LUCA008. Without wishing to be bound by any particular theory, it is believed that such bacteria can ameliorate, prevent, treat, or decrease the likelihood of this outcome by any one or more of (i) increasing the abundance of *Lactobacillus* in and/or decreasing the diversity of the microbiota; and (ii) inhibiting, limiting, or preventing the growth of *Mycoplasma, Ureaplasma,* or other *Mollicutes.*
(7) Miscarriage. Bacteria in the live biotherapeutic formulation used to ameliorate, prevent, treat, or decrease the likelihood of this outcome may comprise a selected strain or consortium of strains comprising at least one of (i) *Lactobacillus crispatus* bacteria configured to express at least one of the asparagine synthase B (*asnB*) gene of SEQ ID NO: 2 and the asparagine synthase B (*asnB*) gene of SEQ ID NO: 3; (ii) *Lactobacillus crispatus* bacteria containing an asparagine synthase B (*asnB*) gene encoding at least one of a polypeptide of SEQ ID NO: 5 and a polypeptide of SEQ ID NO: 6; and (iii) at least one strain selected from the group consisting of LUCA 111, LUCA011, LUCA015, LUCA009, LUCA102, LUCA006, LUCA059, LUCA103, and LUCA008. Without wishing to be bound by any particular theory, it is believed that such bacteria can ameliorate, prevent, treat, or decrease the likelihood of this outcome by any one or more of (i) ameliorating, preventing, treating, or decreasing the likelihood of bacterial vaginosis, which is associated with miscarriage; and (ii) remediating a *Lactobacillus-*poor (CST IV) microbiota; and (iii) inhibiting, limiting, or preventing the growth of, or otherwise decreasing or preventing an increase in the abundance of, *Gardnerella vaginalis.*
(8) Chorioamnionitis and/or intra-amniotic infection. Bacteria in the live biotherapeutic formulation used to ameliorate, prevent, treat, or decrease the likelihood of this outcome may comprise a selected strain or consortium of strains comprising at least one of (i) *Lactobacillus crispatus* bacteria configured to express at least one of the asparagine synthase B (*asnB*) gene of SEQ ID NO: 2 and the asparagine synthase B (*asnB*) gene of SEQ ID NO: 3; (ii) *Lactobacillus crispatus* bacteria containing an asparagine synthase B (*asnB*) gene encoding at least one of a polypeptide of SEQ ID NO: 5 and a polypeptide of SEQ ID NO: 6; and (iii) at least one strain selected from the group consisting of LUCA 111, LUCA011, LUCA015, LUCA009, LUCA102, LUCA006, LUCA059, LUCA103, and LUCA008. Without wishing to be bound by any particular theory, it is believed that such bacteria can ameliorate, prevent, treat, or decrease the likelihood of this outcome by any one or more of (i) ameliorating, preventing, treating, or decreasing the likelihood of bacterial vaginosis, which is associated with chorioamnionitis and intra-amniotic infection; and (ii) remediating a *Lactobacillus-*poor (CST IV) microbiota.
(9) Infertility and/or ineffectiveness of fertility treatment. Bacteria in the live biotherapeutic formulation used to ameliorate, prevent, treat, or decrease the likelihood of this outcome may comprise a selected strain or consortium of strains comprising at least one of (i) *Lactobacillus crispatus* bacteria configured to express at least one of the asparagine synthase B (*asnB*) gene of SEQ ID NO: 2 and the asparagine synthase B (*asnB*) gene of SEQ ID NO: 3; (ii) *Lactobacillus crispatus* bacteria containing an asparagine synthase B (*asnB*) gene encoding at least one of a polypeptide of SEQ ID NO: 5 and a polypeptide of SEQ ID NO: 6; and (iii) at least one strain selected from the group consisting of LUCA 111, LUCA011, LUCA015, LUCA009, LUCA102, LUCA006, LUCA059, LUCA103, and LUCA008. Without wishing to be bound by any particular theory, it is believed that such bacteria can ameliorate, prevent, treat, or decrease the likelihood of this outcome by any one or more of (i) ameliorating, preventing, treating, or decreasing the likelihood of bacterial vaginosis, which is associated with infertility and/or ineffectiveness of fertility treatment; and (ii) decreasing the diversity of the microbiota.
(10) Cervical intraepithelial neoplasia, cervical cancer, and/or another cancer of the female genitourinary system. Bacteria in the live biotherapeutic formulation used to ameliorate, prevent, treat, or decrease the likelihood of this outcome may comprise a selected strain or consortium of strains comprising at least one of (i) *Lactobacillus crispatus* bacteria configured to express at least one of the asparagine synthase B (*asnB*) gene of SEQ ID NO: 2 and the asparagine synthase B (*asnB*) gene of SEQ ID NO: 3; (ii) *Lactobacillus crispatus* bacteria containing an asparagine synthase B (*asnB*) gene encoding at least one of a polypeptide of SEQ ID NO: 5 and a polypeptide of SEQ ID NO: 6; and (iii) at least one strain selected from the group consisting of LUCA 111, LUCA011, LUCA015, LUCA009, LUCA102, LUCA006, LUCA059, LUCA103, and LUCA008. Without wishing to be bound by any particular theory, it is believed that such bacteria can ameliorate, prevent, treat, or decrease the likelihood of this outcome by (i) increasing the abundance of *L. crispatus* and/or providing an *L. crispatus-dominated* (CST I) microbiota; (ii) inhibiting, limiting, or preventing the growth of, or otherwise decreasing or preventing an increase in the abundance of, any one or more of *Atopobium vaginae, Dialister invisus, Finegoldia magna, Gardnerella vaginalis, Peptostreptococcus anaerobius, Porphyromonas uenonis, Prevotella buccalis,* and *Prevotella timonensis;* and (iii) ameliorating, preventing, treating, or decreasing the likelihood of human papillomavirus infection, or reducing the abundance of bacteria associated therewith.

### EXAMPLES

The invention is further described by way of the following non-limiting Examples.

### Example 1: Construction of Human Vaginal Non-Redundant Gene Catalog

This Example describes the construction of a human vaginal non-redundant gene catalog (VIRGO) according to the methods of the present invention.

211 newly sequenced vaginal datasets and 53 vaginal datasets downloaded from the HMP data repository were obtained. Genome sequences of deposited urogenital bacterial isolates were downloaded from multiple databases, including GenBank, Integrated Microbial Genomes & Microbiomes (IMG/M), and the HMP referencing genome database. After removing duplicate genomes under the same strain names, genomes of 322 urogenital bacterial strains representing 152 bacterial species were included.

The 211 newly sequenced metagenomes were generated as follows: whole genomic DNA was extracted from 300 µL aliquots of vaginal ESwab re-suspended into 1 mL of Amies transport medium and preserved at -80 °C. Cells were then lysed using a combination of enzymatic digestion (including mutanolysin, lysostaphin, and lysozyme treatment) and mechanical disruption, followed by proteinase K, SDS, and bead beating steps. DNA extraction and concentration qualification were performed according to the procedures described in Jacques Ravel et al., "Vaginal microbiome of reproductive-age women," 108(S1) Proceeding of the National Academy of Sciences 4680 (June 2010) (hereinafter "Ravel"). The shotgun metagenomic sequence libraries were constructed from the extracted DNA using Illumina Nextera XT kits and sequences on an Illumina HiSeq 2500 platform (150 bp paired end mode, eight samples per lane).

The metatranscriptomes used to demonstrate the use of the present invention for the analysis of community-wide gene expression were obtained from RNA extracted from vaginal swabs stored in 2 mL of Amies Transport Medium-RNAlater solution (50/50 by volume) archived at -80 °C. A total of 500 µL of ice-cold PBS was added to 1,000 µL of that solution and spun at 8,000g for 10 minutes. The pellet was resuspended in 500 µL of ice-cold RNase-free PBS with 10 µL of β-mercaptoethanol. The suspension was transferred to Lysis Matrix B tubes containing 100 µL of 10% SDS and 500 µL of acid phenol and bead beaten using a FastPrep instrument for 45 seconds at 5.5 m/s. The aqueous phase was mixed with 250 µL of acid phenol and 250 µL of a 24:1 solution of chloroform and isoamyl alcohol. The aqueous layer was again transferred to a fresh tube and mixed with 500 µL of the chloroform/isoamyl alcohol solution. For each part by volume of resulting aqueous solution, 0.1 parts of 3 M sodium acetate, 0.01 parts of 5 mg/mL glycogen, and three parts of 100% ethanol were added. The mixture was incubated at -20 °C overnight to precipitate the nucleic acids. After centrifugation at 13,400g for 30 minutes at 4 °C, the resulting pellet was washed, dried, and dissolved in 100 µL of DEPC-treated water. Carryover DNA was removed by (1) treating twice with Turbo DNase free at two half-hour intervals, according to the manufacturer's protocol, for rigorous DNase treatment, and (2) purifying twice using gDNA-eliminator columns before and after DNase treatment, followed by RNeasy column purification. PCR was further conducted using 16S rRNA primer 27F (5'-AGAGTTTGATCCTGGCTCAG-3') and 534R (5'-CATTACCGCGGCTGCTGG-3') to confirm DNA removal. The quality of extracted RNA was checked using an Agilent 2100 Expert Bioanalyzer Nano chip. Ribosomal RNA removal was performed with a combined Gram-positive, Gram-negative, and human/mouse/rat Ribo-Zero rRNA Removal Kit, according to the manufacturer's protocol. The resulting RNA was purified using a Zymo Research RNA Clean & Concentrator-5 column kit. Final RNA quality was checked using an Agilent RNA 6000 Expert Bioanalyzer Pico chip. Sequencing libraries were prepared using an Illumina TruSeq RNA sample prep kit with a modification to the manufacturer's protocol: cDNA was purified between enzymatic reactions and library size selection was performed with AMPure XT beads. Library sequencing was performed using the Illumina HiSeq 2500 platform (150 bp paired end mode, eight samples per lane).

Multiple bioinformatics pre-processing steps were applied to the raw shotgun metagenomic sequence datasets, including (1) eliminating all human sequence reads (including human rRNA LSU/SSU sequence reads) using BMTagger v3.101 against a standard human genome reference (GRCh37.p5, as described in Deanna M. Church et al., "Modernizing reference genome assemblies," 9(7) PLos Biology e1001091 (July 2011)); *(2) in silico* microbial rRNA sequence reads depletion by aligning all reads using Bowtie v1 (as described in Ben Langmead et al., "Ultrafast and memory-efficient alignment of short DNA sequences to the human genome," 10 Genome Biology R25 (Mar. 2009)) against the SILVA PARC ribosomal-subunit sequence database (as described in Christian Quast et al., "The SILVA ribosomal RNA gene database project: improved data processing and web-based tools," 41(D1) Nucleic Acids Research D590 (Jan. 2013)) to eliminate misassemblies of these repeated regions, after each of which steps the paired reads were removed; and (3) stringent quality control using Trimmomatic v0.36 (as described in Anthony M. Bolger et al., "Trimmomatic: a flexible trimmer for Illumina sequence data," 30(15) Bioinformatics 2114 (Aug. 2014)), in which the Illumina adapters were excised, reads were trimmed using a 4bp sliding window with an average quality score threshold of Q15, and reads containing any ambiguous bases were removed. MetaPhlAn v2 (as described in Nicola Segata et al., "Metagenomic microbial community profiling using unique clade-specific marker genes," 9 Nature Methods 811 (June 2012)) was subsequently used to establish taxonomic profiles after these pre-processing steps. Samples were then clustered in community state types (CSTs) using taxa abundance tables and the Jensen-Shannon divergence metrics as described in Ravel. The 264 vaginal metagenomes were then assembled using IDBA-UD v1.0 (as described in Yu Peng et al., "IDBA-UD: a de novo assembly for single-cell and metagenomic sequencing data with highly uneven depth," 28(11) Bioinformatics 1420 (June 2012)) with a k-value range of 20 to 100.

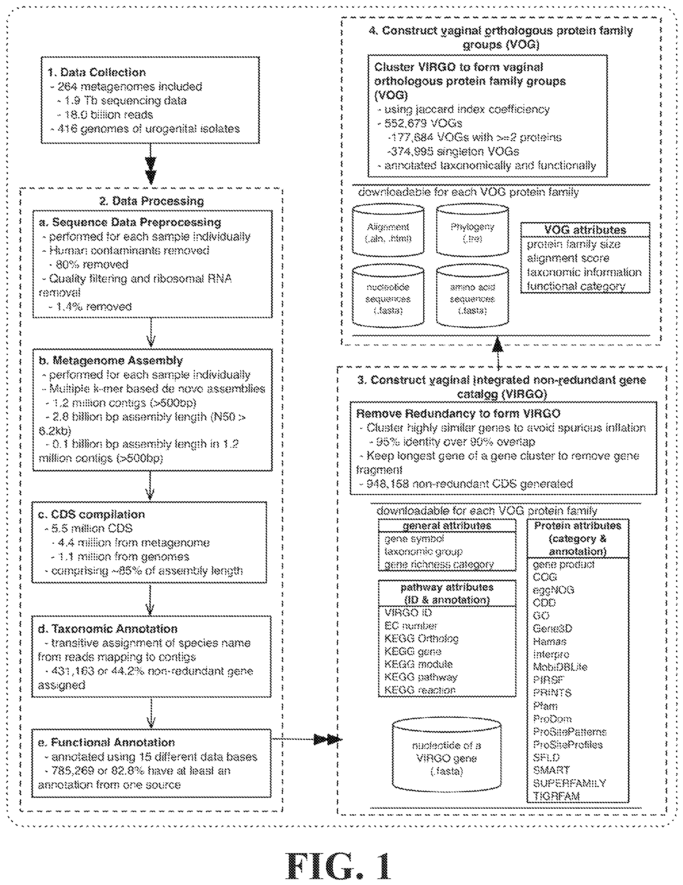

Genes were called on the resulting contigs using MetageneMark v3.25 (as described in Wenhan Zhu et al., "Ab initio gene identification in metagenomic sequences," 38(12) Nucleic Acids Research e132 (July 2010)) to predict coding DNA sequences (CDSs) with the default settings; Figure 1 illustrates the method used to identify and cluster CDSs. Metagenomic assemblies contributed about 80% of the CDSs, while the remaining about 20% originated from urogenital bacteria isolate genome sequences. Genes and gene fragments that were at least 99bp long, with greater than 95% identity over 90% of the shorter gene length, were clustered together by a greedy pairwise comparison implemented in CD-HIT-EST v4.6 (as described in Weizhong Li et al., "Clustering of highly homologous sequences to reduce the size of large protein databases," 17(3) Bioinformatics 282 (Mar. 2001)), according to the clustering procedure and threshold described in Junjie Qin et al., "A human gut microbial gene catalogue established by metagenomic sequencing, 464 Nature 59 (Mar. 2010) (hereinafter "Qin"), and Junhua Li et al., "An integrated catalog of reference genes in the human gut microbiome," 32 Nature Biotechnology 834 (July 2014). The gene with the longest length greater than or equal to 99bp was used as the representative for each cluster of redundant genes. This process afforded the removal of partial genes and eliminated overcalling as unique because of sequencing errors.

A total of 948,158 non-redundant CDSs longer than 99 bp were identified and retained, representing 17.2% of the original 5.5 million CDSs. The newly sequenced vaginal metagenomes used to build VIRGO contributed 12 times more non-redundant genes (634,288 genes) than the HMP vaginal metagenomes (54,500 genes). Combined, the metagenomes contributed twice as many non-redundant genes as urogenital bacterial isolate genome sequences.

Of the approximately 18 billion reads generated for the newly sequenced metagenomes, about 14.4 billion (79.7%) were identified as human sequences and removed, but the present inventors found that vaginal metagenomes dominated by *Lactobacillus* spp. had significantly higher proportions of human sequence reads than those from *Lactobacillus-deficient* metagenomes (88.7% vs. 73.3%, *t* = -6.6, *P* < 0.001). The newly sequenced metagenomes totaled 1.2 million contigs, with a combine length of 2.8 billion base pairs and an N₅₀ of 6.2 kbp. The metagenomic data obtained from the HMP contributed 40,000 contigs, comprising 100 million bp of assembled sequence; the newly sequenced metagenomes provided 19.5 times more assembled length than the HMP vaginal metagenomes.

The MetaPhlAn taxonomic analysis of the metagenomes revealed that the microbial communities contained 312 bacterial species present at a relative abundance of at least 0.01%. Among others, all major vaginal *Lactobacillus* species (*L. crispatus, L. gasseri, L. iners,* and *L. jensenii*)*,* as well as common facultative and strict anaerobic vaginal species (e.g. *Gardnerella vaginalis, Atopobium vaginae, Prevotella amnii, Megasphaera* genomosp., *Mobiluncus mulieris, Mageebacillus indolicus* (BVAB3), and *Veillonella parvula*) were identified in the metagenomes., Even bacteria associated with bacterial vaginosis that are often only present at low abundance-e.g. *Finegoldia magna, Peptoniphilus harei, Peptostreptococcus anaerobius, Mobiluncus curtisii, Peptoniphilus lacrimalis, Anaerococcus tetradius, Ureaplasma urealyticum, Veillonella atypica,* and *Corynebacterium glucuronolyticum-*were represented. The taxonomic profiles of 264 metagenomes encompassed the five vaginal community state types (CSTs) reported in Ravel, with frequencies of 18.9% for CST I, 3.8% for CST II, 20.5% for CST III, 48.5% for CST IV, and 8.3% for CST V. These results highlight the taxonomic breadth of the vaginal bacterial communities included in the construction of VIRGO.

### Example 2: Bioinformatics Analysis

This Example demonstrates the comprehensiveness of reference gene catalogs generated according to the present invention.

The comprehensiveness of VIRGO was tested using vaginal metagenomics data from 91 vaginal metagenomes obtained from North American women not included in the construction of VIRGO or sequenced in this study, as well as African and Chinese women, which allowed for determination of the utility of VIRGO to analyze metagenomes from other populations. The sequence reads were first mapped to the VIRGO contigs using Bowtie v2 (parameters --threads 4 --sensitive-local -D 10 -R 2 -N 0 -L 22 -i S,1,1.75 -k 1 --ignore-quals --no-unal, as described in Ben Langmead and Steven L. Salzberg, 9 Nature Methods 357 (Mar. 2012)), according to the criteria described in Qin. Any unmapped reads were compared to the GenBank nt database (as described in NCBI Resource Coordinators, "Database resources of the National Center for Biotechnology Information," 45(Database) Nucleic Acids Research D12 (Jan. 2017)), using BLASTN and an E-value of 1E-10 as cutoff. To annotate BVAB1 genes in VIRGO, BLASTN and an E-value of 1E-10 as cutoff were likewise used, and the matched genes with more than 95% identity over more than 90% of gene length were annotated as BVAB1 genes. To retrieve pullulanase (*pulA*) genes in VIRGO, conserved protein domain CDD annotation (as described in Aron Marchler-Bauer et al., "CDD: NCBI's conserved domain database," 43(D1) Nucleic Acids Research D222 (Jan. 2015) (hereinafter "Marchler-Bauer")) was used with keyword "pullulanase." To further demonstrate the comprehensiveness of VIRGO and the fact that VIRGO captures the pangenome of selected species, species-specific metagenome accumulation curves and diversity estimates for the number of non-redundant genes were constructed by rarefaction with 100 bootstraps using R packages *iNEXT* v2.0 and *vegan* v2.5-5 (as described in Philip Dixon, "VEGAN, a package of R functions for community ecology," 14(6) Journal of Vegetation Science 927 (Dec. 2003)) for seven species: *A. vaginae, G. vaginalis, L. crispatus, L. gasseri, L. iners, L. jensenii,* and *P. timonensis.*

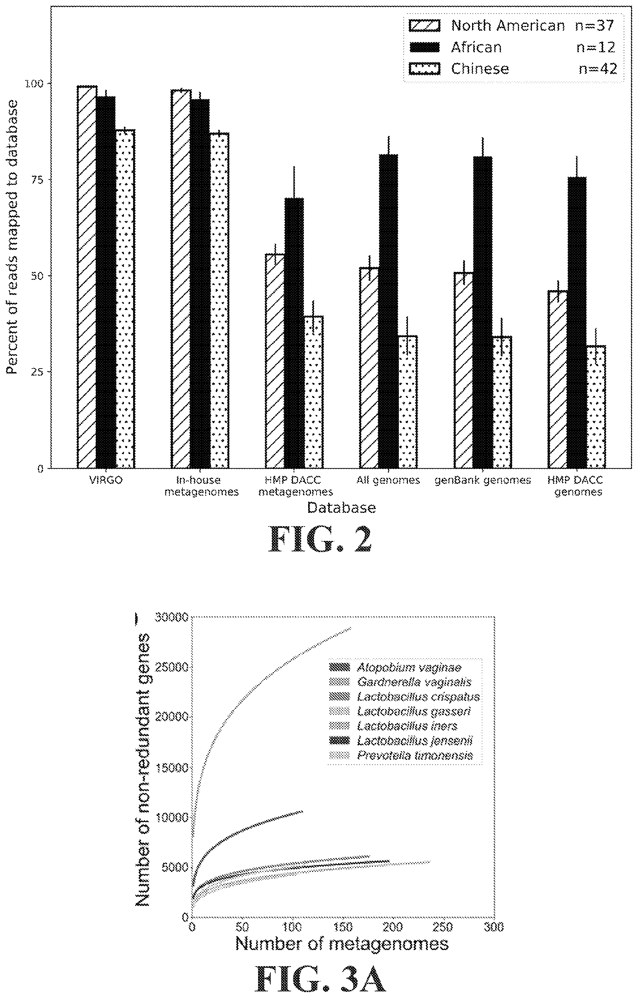

As illustrated in Figure 2, more than 99% of the reads from North American metagenomes were able to be mapped to the complete VIRGO dataset, whereas only about 55% of these reads mapped to contigs from the HMP vaginal metagenomes subset. This result indicates a lack of genetic diversity in the HMP vaginal metagenomes, which were derived from highly selected and otherwise healthy women. Further, despite originating from populations not used in the construction of VIRGO, 96% of the reads from African women and 88% of the reads from Chinese women mapped to the complete VIRGO dataset. For these two cohorts 71.7% and 99.9%, respectively, of the reads that failed to map to VIRGO also did not have a match in GenBank.

By including many metagenomes and bacterial isolate genome sequences, each vaginal species' pangenome is represented in VIRGO. The extent of this representation is illustrated in the metagenome accumulation curves for the seven key vaginal species identified above, as shown in Figure 3A. These curves track the number of new non-redundant genes added when increasing numbers of metagenomes containing a given species are included in constructing the database. The accumulation curves for six of the seven species (all except *G. vaginalis*)*,* indicating that VIRGO includes the majority of these species' pangenomes. The number of non-redundant genes for five of the six species are similar (about 5,000 genes), while for *A. vaginae* the number is roughly twice this amount. These gene counts pale in comparison, however, to the number of non-redundant genes included in VIRGO for *G. vaginalis,* which surpasses 25,000 genes. These results illustrate the comprehensiveness of VIRGO and its broad application to different populations and ethnicities.

### Example 3: Taxonomic and Functional Annotation of VIRGO

This Example demonstrates the utility of reference gene catalogs produced according to the present invention to characterize vaginal microbial communities.

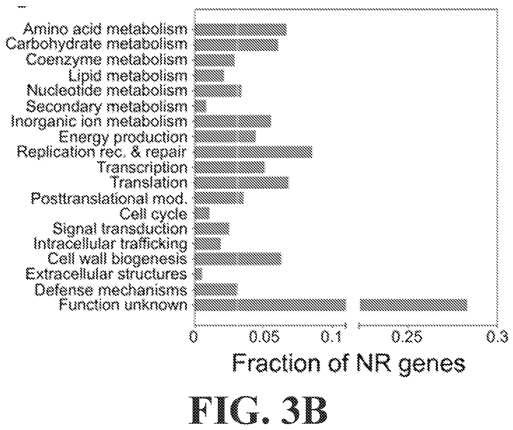

The non-redundant genes of VIRGO were annotated with a rich set of taxonomic and functional information. Genes that originated from an isolate sequence genome were automatically assigned that species name. For metagenomes, taxonomy was assigned to a metagenomic contig by mapping the sequence reads making up that contig to the Integrated Microbial Genomes (IMG) reference database (v400) using Bowtie v1 (parameters: "-l 25 --fullref --chunkmbs 512 --beststrata -m 20"). A secondary filter was applied so that the total number of mismatches between the read and the reference was less than 35 and the first 25 bp of the read matched the reference. Using the results of this mapping, taxonomy was assigned to all genes encoded on the contig that matched the following four criteria: (1) at least 95% of the reads mapped to the same bacterial species, (2) the remaining 5% of off-target reads did not map to a single species, (3) the contig had at least 2x average coverage and more than 50 reads, and (4) at least 25% of the total length of the contig had reads mapped thereon. These stringent criteria were used to ensure high fidelity of the taxonomic assignments and a low contribution of potentially chimeric contigs. To further diminish the risk of incorporating false taxonomic assignments, the annotations of the contigs belonging to species at low relative abundance in the sample were removed. Genome completeness was estimated as the fractional representation of the genome in the metagenome using BLASTN (minimal overlapping of more than 60% of the shorter sequence and more than 80% sequence similarity). For each metagenome, only taxonomic assignments originating from species with at least 80% representation were incorporated. The genes that showed more than 80% sequence similarity to the non-redundant genes over 60% of query gene length were then assigned. The non-redundant genes in VIRGO were searched against a fungal database that included five vaginal yeast species in 40 genomes using BLASTN, such that a gene must have at least 80% sequence similarity over 60% of overlapping length to be curated. Potential phage genes that may be present in VIRGO were also annotated by searching against phage orthologous groups or prokaryotic virus orthologous groups (version 2016, as described in David M. Kristensen et al., "Orthologous gene clusters and taxon signature genes for viruses of prokaryotes," 195(5) Bacteriology 941 (Mar. 2013)), using BLASTN and including the genes having more than 80% sequence similarity over 60% of query gene length in annotation. Functional annotations were based on the standard procedure for each of 17 functional databases, including cluster of orthologous groups (COG as described in Tatusov, eggnog (v4.5) as described in Huerta-Cepas, and KEGG as described in Kanehisa), conserved protein domain (CDD as described in Marchler-Bauer, Pfam as described in Finn, ProDom as described in Bru, PROSITE as described in Sigrist, TIGRFAM as described in Haft, and InterPro as described in Hunter), domain architectures (CATH-Gene3D as described in Lam and SMART as described in Letunic), intrinsic protein disorder (MobiDB as described in Potenza), high-quality manual annotation (HAMAP as described in Petruzzi), protein superfamily (PIRSF as described in Nikolskaya), a compendium of protein fingerprints (PRINTS as described in Attwood), and gene product attributes (Gene Ontology and JCVI SOP as described in Tanenbaum). An overview of the eggNOG functions encoded in VIRGO is shown in Figure 3B.

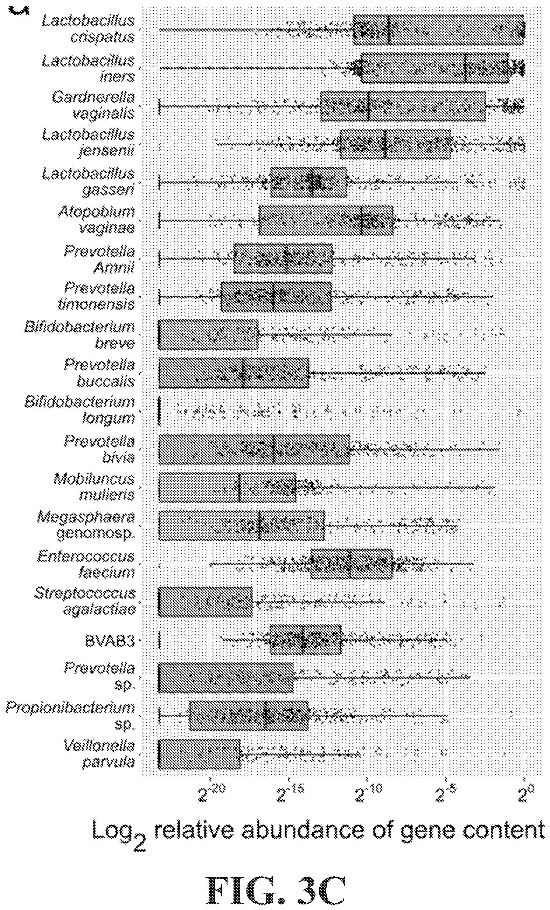

A total of 445,739 non-redundant genes, comprising 47.0% of VIRGO, were able to be taxonomically annotated. Overall, 271 unique bacterial species were annotated in VIRGO, representing a majority of the described vaginal species. This includes BVAB1, a currently unculturable vaginal species, for which a closed genome and several metagenome-assembled genomes (MAGs) have recently been made available. When stratified by CST, CST IV metagenomes have the smallest proportion (less than 30%) of their gene content taxonomically annotated, compared to about 45% to 50% in *Lactobacillus-*dominated CSTs. The most abundant species based on gene content are shown in Figure 3C. The curated potential fungal and phage genes were generally present in low abundance (0.17% ± 0.04% and 0.03% ± 0.001%, respectively). An additional 10,908 fungal genes and 15,965 phage genes were included.

Overall, 785,268 genes-82.8% of all non-redundant genes-were assigned a functional annotation from at least one source. This gene-rich annotation of the non-redundant gene catalog enables a comprehensive functional characterization of vaginal metagenomes and metatranscriptomes.

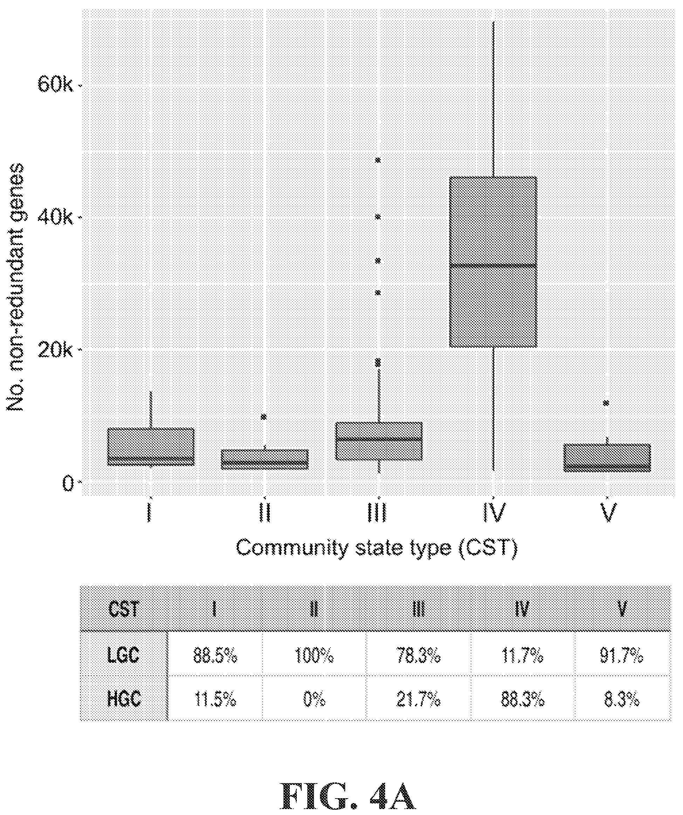

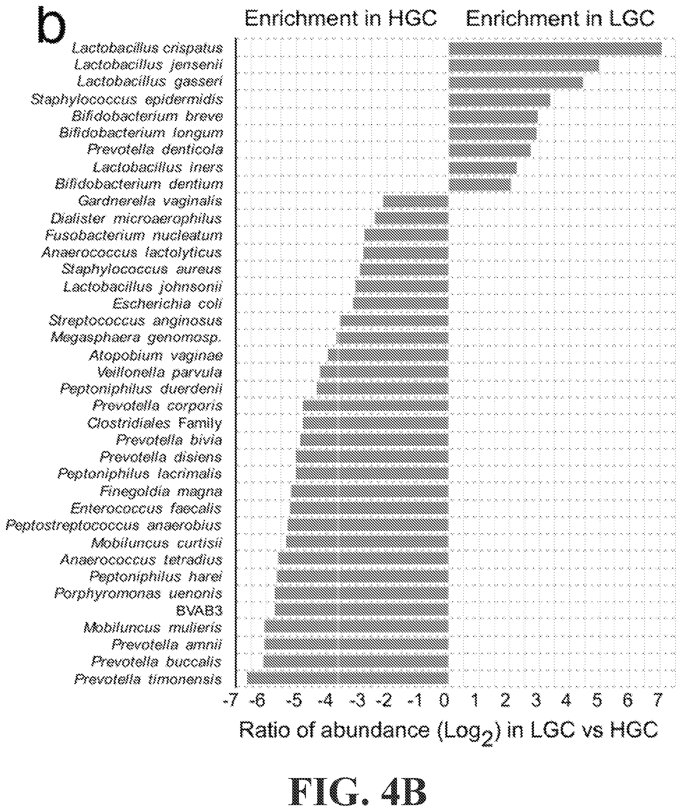

The community gene content, or gene richness, can be characterized as the number of non-redundant genes. As illustrated in Figure 4A, *Lactobacillus-*dominated communities were typically categorized as low gene count (LGC), as 82.9% of these communities have fewer than 1,000 genes; *Lactobacillus-*deficient communities commonly have high gene count (HGC), in that 88.3% of these communities have more than 1,000 genes. Further, genes of a particular vaginal species can be overrepresented in HGC or LGC vaginal communities (Figure 4B) with distinct functional makeups. *Lactobacillus* spp., particularly *L. crispatus, L. jensenii, L. gasseri,* and *L. vaginalis,* were observed to be highly overrepresented in LGC communities. On the other hand, genes belonging to many other species associated with bacterial vaginosis, particularly *P. timonensis, P. buccalis, P. amnii, M. mulieris, Mageeibacillus indolicus, Porphyromonas uenonis, P. harei, Anaerococcus tetradius,* and *M*. *curtisii,* were overrepresented in HGC communities. These results illustrate that gene richness-based annotations can provide an added dimension to the understanding of the genetic basis of the biological processes that drive vaginal microbiomes.

### Example 4: Construction of Vaginal Orthologous Groups for Protein Families

This Example illustrates clustering of genes in reference gene catalogs according to the present invention based on orthology to generate a set of orthologous groups, in this case vaginal orthologous groups (VOGs).

A modified version of a Jaccard clustering method, as previously implemented in David R. Riley et al., "Using Sybil for interactive comparative genomics of microbes on the web," 28(2) Bioinformatics 160 (Jan. 2012), was used to cluster genes into VOGs. An all-against-all BLASTP search was performed among the translated coding sequences (CDS) of the non-redundant genes included in VIRGO. The all-against-all BLASTP matches were used to compute a Jaccard similarity coefficient for each pair of translated CDSs, without constraints based on the sample or organism from which it originated. Only BLASTP matches with at least 80% sequence identity, at least 70% overlap, and an E-value of less than 1E-10 were used in the calculation of the Jaccard similarity coefficient. The filtered BLASTP results were then used to define connections between pairs of translated CDSs, resulting in a network graph with the translated CDSs as nodes and their connections as edges. The Jaccard similarity coefficient was then calculated as the number of nodes that had direct connections to the two translated CDSs divided by the total number of nodes that had direct connections to either of the two translated CDSs in the network (intersection divided by union, as described in Jonathan Crabtree et al. "Sybil: methods and software for multiple genome comparison and visualization," in Michael F. Ochs (ed.), Gene Function Analysis 93 (2007)). A Jaccard cluster (JACs) was defined as a set of translated CDSs whose Jaccard similarity coefficient was at least 0.55. If two translated CDSs from different JACs were reciprocal best matches according to the BLASTP searches, the two JACs were merged and defined as Jaccard orthologous clusters (JOCs). Finally, the alignment program T-Coffee (as described in Cédric Notredame, "T-coffee: a novel method for fast and accurate multiple sequence alignment," 302(1) Journal of Molecular Biology 205 (Sep. 2000)) was used to assess the alignment quality within the JACs and to calculate the alignment score.

The JOCs (orthologous protein families) can be highly conserved (having an alignment score of more than 950) or partially aligned with both conserved and variable regions (having an alignment score of about 300). This result highlights the flexibility of the network-based aggregation algorithm used to recruit both highly similar and distantly related proteins without imposing a single similarity threshold. A total of 617,127 JACs and 552,679 JOCs were generated, of which 177,684 JOCs contained at least two genes while the remaining 374,995 JOCs were singletons, indicating that 38.5% of all VOG proteins are unique.

To demonstrate the utility of VOGs, 32 proteins of the orthologous family encoding vaginolysin, a *G. vaginalis* cholesterol-dependent cytolysin that is key to its pathogenicity as it forms pores in epithelial cells, were retrieved. Using the retrieved alignment, three amino acid variants in an 11-amino acid sequence of domain 4 of vaginolysin were identified. One of the three variants, an alanine-to-valine substitution that is divergent across *G. vaginalis,* had not been reported previously. Thus, VOGs can be mined to understand biological relevance-in this case, potential differences in pore formation activity and possibly cytotoxicity, which can be further investigated and possibly exploited to formulate live biotherapeutics. As another non-limiting example, VOGs were searched using the key phrase "cell surface-associated proteins" and "L. *iners"* and retrieved two protein families, one of which was recognized to have an LPXTG motif while the other harbored the motif YSIRK. Notably, a previous study on staphylococcal proteins suggested that the motifs LPXTG and YSIRK are involved in different biological processes related to surface protein anchoring to the cell wall envelope. The two retrieved protein families are specific to *L. iners* and provide a relevant starting point for further investigation of its adherence and/or formulation of live biotherapeutics leveraging this difference. These results demonstrate how a VOG database generated according to the present invention can be used to explore and exploit more mechanistic understandings of vaginal bacterial communities.

### Example 5: Integration of Metagenome and Metatranscriptome Data

This Example illustrates how reference gene catalogs generated according to the present invention enable the characterization and integrative analysis of the abundance of genes and their expression in a microenvironment, in this case the vaginal microenvironment.

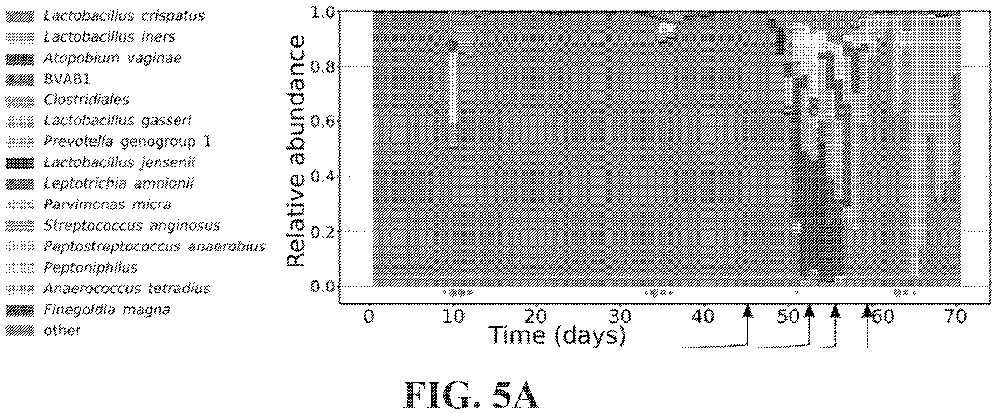

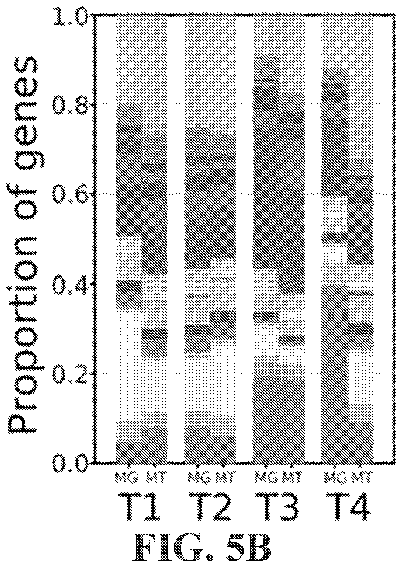

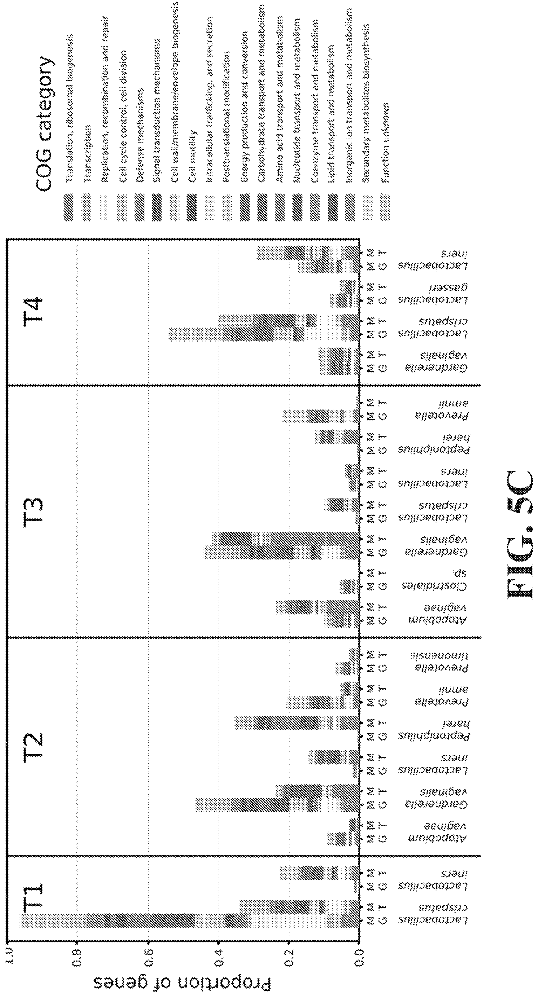

A female human subject's vaginal metagenomes and associated metatranscriptomes were analyzed at four time points: prior to (T1), during (T2 and T3), and after (T4) an episode of symptomatic bacterial vaginosis, as illustrated in Figure 5A (the arrows represent, from left to right, the time points T1-T4). Unsurprisingly, the expressed functions represented in the metatranscriptomes were often different from the encoded functional makeup of the corresponding metagenomes, as illustrated in Figure 5B. VIRGO provided rapid binning of genes by species, which revealed dramatic differences in gene abundance and their transcriptional activity in vaginal species, as illustrated in Figure 5C. Prior to the episode of bacterial vaginosis (time point T1), a small proportion (1.5%) of *L. iners* genes were present, but these genes exhibited high expression levels, accounting for over 20% of the metatranscriptome. At the same time point, *L. crispatus* genes made up the majority of the genes present (96.3%) but exhibited low expression levels (34.2% of the metatranscriptome). By contrast, near the end of the episode of bacterial vaginosis (time point T3), *L. crispatus* genes made up a small proportion of the metagenome but were highly transcriptionally active. This increased activity corresponded with *L. crispatus* regaining dominance at T4, following the resolution of the episode of bacterial vaginosis. Notably, the functions encoded by *G. vaginalis* were similar between T2 and T3, but their expression differed between these time points. By enabling this integration of these types of data, reference gene catalogs generated according to the present invention can thus provide a functional understanding of the microbiota, e.g. the vaginal microbiota, and provide insight into the formulation of appropriate live biotherapeutics for the treatment of a particular disease or disorder associated with specific metagenomic and/or metatranscriptomic characteristics.

### Example 6: Characterization of Within-Community Intraspecies Diversity

This Example illustrates how reference gene catalogs generated according to the present invention can be used to conduct intraspecies diversity analyses.

Intraspecies diversity analyses were conducted by mapping isolate genome sequences and vaginal metagenomes to VIRGO. The analysis was focused on the seven vaginal species discussed in Example 2 above. A total of 1,507 vaginal metagenomes, including 1,403 metagenomes newly obtained from de-identified vaginal swab and lavage specimens and 76 publicly available metagenomes, were mapped against VIRGO. For each of the seven bacterial species, a presence/absence matrix for the species' non-redundant genes, including the data from the species' isolate genomes and all metagenomes that contained at least 80% of the average number of genes encoded on a genome of that species, was constructed. Comparisons of the number of non-redundant genes present in the species isolate genomes against the metagenomes in which they appeared were conducted using the student t-test. Hierarchical clustering was performed on the Boolean matrix of the species' non-redundant genes using Jaccard clustering implemented in the *vegan* package in R.

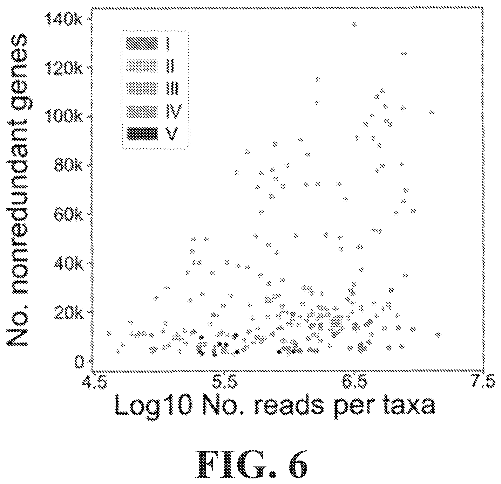

The number of non-redundant genes identified in a metagenome was not found to correlate with the depth sequencing, as illustrated in Figure 6. Most of each species' gene content was recovered even when that species was present at low abundance (less than 1%) in a community. For instance, even though *P. timonensis* was generally present in low abundance in these metagenomes (mean 4.8%, standard deviation 0.3%, minimum 0.1, maximum 33.8%), the majority of its genome was recovered (2,469 ± 401 CDSs). Similarly high sensitivity was observed in the analysis of the other six selected vaginal species. These results demonstrate the capability of reference gene catalogs generated according to the present invention to characterize the gene content of even low-abundance taxa from metagenomics data.

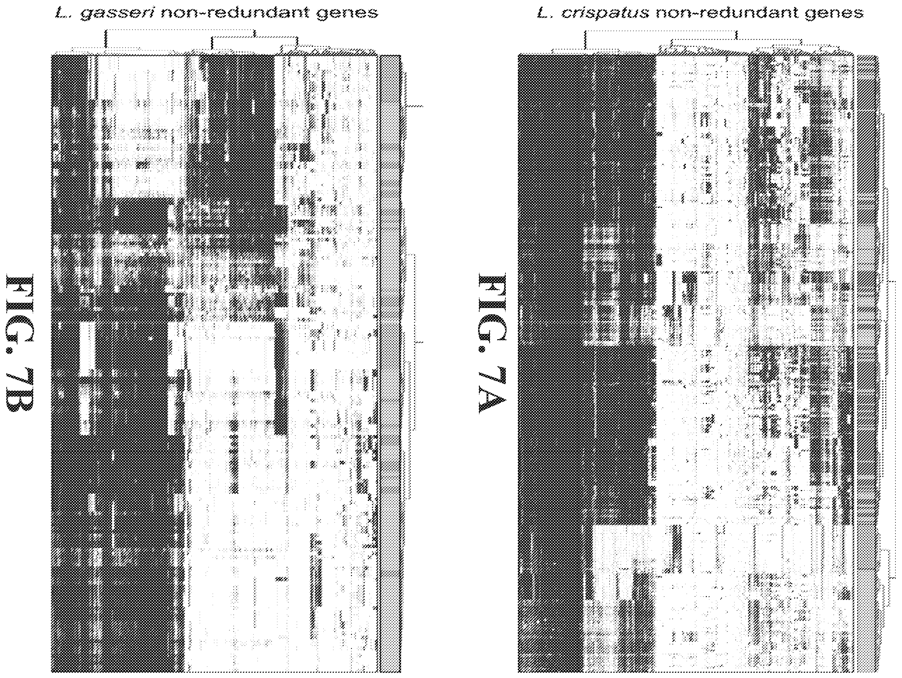

Using these species-specific gene repertoires, the amount of intraspecies diversity present within an individual woman's vaginal microbiome can be characterized. Because VIRGO (or another reference gene catalog of the invention) comprises the "pangenomes" of each vaginal bacterial species, it can be used to evaluate the amount of intraspecies diversity present in microbiome communities. The number of genes that were assigned to each of the seven species in each of the 1,507 metagenomic datasets were counted and compared to the number of genes found in each species' reference genome. The number of genes for a species in a community often exceeded the number found in a single isolate genome, as illustrated in Figures 6A and 6B, suggesting that multiple strains of a species co-occur in vaginal bacterial communities. The total number of *L. crispatus* genes identified in each of the metagenomes where it was detected contained, on average, 1.6 times more genes (3,262 ± 586) than were found encoded on *L. crispatus* genomes (2,064 ± 225, *P* < 0.001). Similar results were observed for *G. vaginalis, A. vaginae, L. iners, L. jensenii,* and *L. gasseri.* Among these species, *G. vaginalis* and *A. vaginae* exhibited the highest degree of intraspecies diversity, while *L. crispatus* had the highest within-metagenome intraspecies diversity among the major *Lactobacillus* spp., as illustrated in Figure 7A. These results suggest that an individual woman's vaginal bacterial population routinely comprises more than one strain of most species, and indicates that reference gene catalogs generated according to the present invention enable the investigation of this intraspecies diversity and/or leveraging of such diversity to formulate live biotherapeutics.

Well-established practices from pangenomics, e.g. the procedures described in Hervé Tettelin et al., "Genome analysis of multiple pathogenic isolates of Streptococcus agalactiae: implications for the microbial 'pan-genome,'" 102(39) Proceedings of the National Academy of Sciences 13950 (Sep. 2005), were applied to identify "core" and "accessory" non-redundant genes among the sample-specific species gene repertoires. Based on the clustering patterns of gene prevalence profiles, groups of consistently present ("core") and variably present ("accessory") non-redundant genes could be defined. The majority of the observed genes for each of the species were categorized as "accessory," with variable representation across the datasets. Using *L. crispatus* as an example, more than twice as many non-redundant genes were observed to have variable representation across the metagenomes than were present in every sample, as illustrated in Figure 7A. Notably, it is clear from this analysis that the gene content identified with VIRGO in genome sequences of *L. crispatus* underrepresent the intraspecies genetic diversity present in the metagenomes. Similar results were observed for the other six species analyzed, although the magnitude of the difference between the metagenome and isolate gene repertoires varied depending on the species. Overall, VIRGO revealed that metagenomic data carry a more extensive gene content than is found in all combined isolate genome sequences.

### Example 7: Metagenomic Subspecies in Vaginal Ecosystem

This Example illustrates how reference gene catalogs generated according to the present invention can be used to identify metagenomic subspecies ("MG-subspecies").

Hierarchical clustering of the metagenome species-specific gene content profiles revealed distinct groupings, defined herein as metagenomic subspecies or MG-subspecies. These metagenomic subspecies represent types of bacterial populations that share a similar gene pool as assessed by shotgun metagenomic sequence data. For example, this analysis revealed at least three distinct metagenomic subspecies for *L. gasseri,* as illustrated in Figure 7B. *L. gasseri* MG-subspecies I and III have large sets of non-redundant genes that are present in one but not the others, while *L. gasseri* MG-subspecies II carries a blend of the genes from both MG-subspecies I and III. Analysis of G. *vaginalis* revealed at least five MG-subspecies, concordant with previous studies that had identified multiple clades within the species. However, it was also found that the genome-based paradigm largely underrepresents the diversity of G. *vaginalis* gene content that was identified in metagenomes. The foregoing analysis was applied to seven vaginal species, and it was found that vaginal microbial communities are often composed of complex mixtures of multiple strains of the same species, and that these mixtures can be clustered into distinct MG-subspecies. Reference gene catalogs generated according to the present invention thus enable investigation of MG-subspecies in the human microbiome and their gene contents, which in turn can reveal novel features of the microbiome and sub-populations thereof that allow for, e.g., selection, tuning, and/or optimization of strains for use in live biotherapeutic formulations.

### Example 8: Gene-Specific Effect of Microbial Species on Microbiome Health

This Example illustrates the use of reference gene catalogs generated according to the present invention to formulate live biotherapeutics.

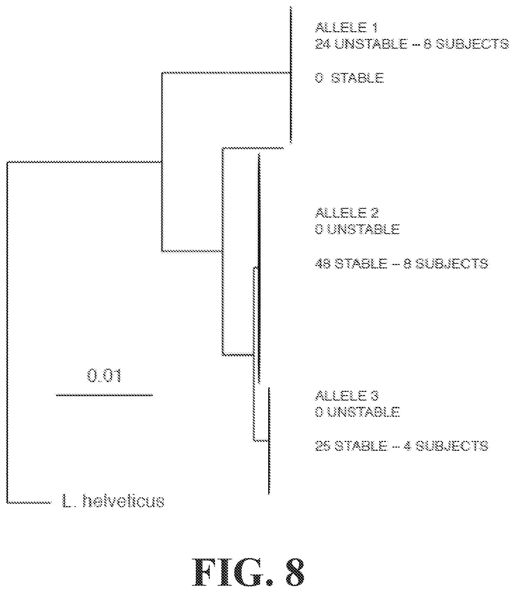

The use of VIRGO revealed a stability pattern of the microbiota from which isolates of *Lactobacillus crispatus* were cultured. Specifically, using a DBGWAS method (as described in Magali Jaillard et al., "A fast and agnostic method for bacterial genome-wide association studies: bridging the gap between k-mers and genetic events," 14(11) PLoS Genetics e1007758 (Nov. 2018)) in conjunction with VIRGO, it was discovered that sequence variants of the asparagine synthase B *(asnB)* gene of *L. crispatus* were strongly associated with stability of *L. crispatus* in the vaginal environment, as illustrated in Figure 8.

Metabolomics have indicated that asparagine synthase can synthesize asparagine from aspartate and glutamine (in which case glutamate is also produced) and/or from aspartate and ammonia. As has been demonstrated previously, e.g. in Pybus, two pathogenic bacteria (G. *vaginalis* and *P. bivia*) associated with bacterial vaginosis and other adverse health outcomes (e.g. inflammation, preterm birth, increased risk of STI) rely on an ammonia-based cycle to flourish in the vaginal environment. Thus, the ability of *L. crispatus* to sequester ammonia in the vagina may break this cycle and slow or prevent the growth of G. *vaginalis* and *P. bivia.* Without wishing to be bound by any particular theory, it is believed that the identified sequence variants in the *asnB* gene of *L. crispatus* may affect glutamine binding and thus production of asparagine by the ammonia sequestration mechanism, thereby preventing the growth of the pathogenic species and resulting in a more stable vaginal environment.

In addition to G. *vaginalis* and *P. bivia,* another common pathogen in the vaginal environment is uropathogenic *Escherichia coli* (UPEC). UPEC causes 80% of all UTI, which affects over ten million women in the United States alone each year, is highly recurrent, and is often highly antibiotic-resistant. UPEC is known to reside in the intestinal tract, but also in the vaginal tract, where it can infect the urethra and ultimately the bladder. Recurrent UTI is an unaddressed healthcare need, and live biotherapeutics that are effective against UPEC are therefore highly desirable.

Three strains of *L. crispatus* having the stability-associated *asnB* gene (identified herein as LUCA015, LUCA011, and LUCA09) were parallel streak assayed against UPEC, clinical *P. bivia,* and clinical G. *vaginalis* on MRS agar + 1% starch. After 24 hours, the inhibiting effects of each strain against each pathogen were scored. The results of the assay are given in Table 2.

**Table 2**

| | ***E. coli* CFT073** | ***P. bivia* C0046E2** | ***G. vaginalis* C0047B2** |
|---|---|---|---|
| ***L. crispatus* LUCA015** | +++ | +++ | +++ |
| ***L. crispatus* LUCA011** | +++ | +++ | +++ |
| ***L. crispatus* LUCA009** | +++ | +++ | +++ |

| | | | |
|---|---|---|---|
| Legend: - = no apparent inhibition; + = about 25% inhibition; ++ = about 50% inhibition; +++ = about 75% inhibition; ++++ = apparently complete inhibition | | | |

These results indicate that the effectiveness of live biotherapeutic formulations can be greatly enhanced by gene-specific selection of bacterial strains included in the live biotherapeutic formulation.

### Example 9: Formulation of Gene-Specific Live Biotherapeutic

This Example further investigates the antimicrobial effect of particular *L. crispatus* strains on common vaginal pathogens.

Using the techniques described herein, nine strains of *L. crispatus* were identified as having a stability-associated *asnB* sequence variant, as described in the preceding Example. Aliquots of these strains were prepared according to Table 3.

**Table 3**

| **ID** | **Strain** | **CFU/mL** |
|---|---|---|
| 1 | LUCA111 | 7.60 · 10⁷ |
| 2 | LUCA011 | 7.00 · 10⁸ |
| 3 | LUCA015 | 2.16 · 10⁸ |
| 4 | LUCA009 | 5.18 · 10⁸ |
| 5 | LUCA102 | 3.80 · 10⁷ |
| 6 | LUCA006 | 9.00 · 10⁷ |
| 7 | LUCA059 | 2.18 · 10⁸ |
| 8 | LUCA103 | 2.10 · 10⁸ |
| 9 | LUCA008 | 1.54 · 10⁸ |

Consortia, or "cocktails," comprising mixtures of four strains were also prepared according to Table 4. (In the table, "strain 1" refers to the most abundant strain in the consortium, "strain 2" to the second-most abundant strain, and so on.)

**Table 4**

| **Consortium ID** | **Strain 1 ID** | **Strain 2 ID** | **Strain 3 ID** | **Strain 4 ID** | **CFU/mL** |
|---|---|---|---|---|---|
| A | 1 | 7 | 3 | 9 | 4.74 · 10⁸ |
| B | 2 | 4 | 3 | 8 | 5.20 · 10⁷ |
| C | 2 | 4 | 3 | 7 | 4.50 · 10⁸ |
| D | 7 | 4 | 3 | 5 | 5.72 · 10⁸ |
| E | 9 | 4 | 3 | 8 | 5.40 · 10⁸ |
| F | 6 | 3 | 4 | 2 | 5.48 · 10⁸ |
| G | 7 | 3 | 6 | 9 | 4.08 · 10⁸ |
| H | 7 | 6 | 5 | 9 | 1.10 · 10⁸ |

Each of the pure strains and consortia of strains was parallel streak assayed against three vaginal pathogens on each of pure MRS agar and MRS agar + 1% starch. After 24 hours, the inhibiting effects of each strain against each pathogen were scored. The results of the assay are given in Table 5.

**Table 5**

| **ID** | **MRS** | | | **MRS + 1% starch** | | |
|---|---|---|---|---|---|---|
| | ***Prevotella* C0117C5** | **UPEC CT131** | **UPEC CT073** | ***G. vag.* ATCC** | ***G. vag.* C0056B5** | **UPEC CT131** |
| 1 | ++ | ++++ | ++++ | ++ | +++ | ++++ |
| 2 | ++ | ++++ | ++++ | ++ | ++++ | ++++ |
| 3 | ++ | ++++ | ++++ | ++ | +++ | ++++ |
| 4 | ++ | ++++ | ++++ | ++ | +++ | ++++ |
| 5 | + | ++++ | ++++ | + | ++ | ++++ |
| 6 | ++ | ++++ | ++++ | ++ | +++ | ++++ |
| 7 | ++ | ++++ | ++++ | - | + | +++ |
| 8 | +++ | ++++ | ++++ | +++ | ++++ | ++++ |
| 9 | ++ | ++++ | ++++ | ++ | +++ | ++++ |
| A | +++ | ++++ | ++++ | +++ | ++++ | ++++ |
| B | +++ | ++++ | ++++ | ++++ | ++++ | ++++ |
| C | +++ | ++++ | ++++ | +++ | ++++ | ++++ |
| D | ++ | ++++ | ++++ | +++ | ++++ | ++++ |
| E | ++++ | ++++ | ++++ | ++ | +++ | ++++ |
| F | ++ | ++++ | ++++ | + | ++ | ++++ |
| G | ++ | ++++ | ++++ | + | +++ | ++++ |
| H | ++ | ++++ | ++++ | ++ | ND | ++++ |

| | | | | | | |
|---|---|---|---|---|---|---|
| Legend: - = no apparent inhibition; + = about 25% inhibition; ++ = about 50% inhibition; +++ = about 75% inhibition; ++++ = apparently complete inhibition | | | | | | |

These results indicate that the effectiveness of live biotherapeutic formulations can be greatly enhanced by gene-specific selection of bacterial strains included in the live biotherapeutic formulation, and particularly by gene-specific selection of consortia of two or more bacterial strains.

The invention illustratively disclosed herein suitably may be practiced in the absence of any element which is not specifically disclosed herein.

## Claims

1. A remedial live biotherapeutic formulation for use in medicine, comprising bacteria adapted to remedy a deficiency or excess of at least one bacterial strain in the subject's vaginal microbial community, and wherein the bacteria comprise a selected strain or consortium of strains comprising at least one of:
(i) *Lactobacillus crispatus* bacteria configured to express at least one of the asparagine synthase B *(asnB)* gene of SEQ ID NO: 2 and the asparagine synthase B *(asnB)* gene of SEQ **ID** NO: 3; and
(ii) *Lactobacillus crispatus* bacteria containing an asparagine synthase B *(asnB)* gene encoding at least one of a polypeptide of SEQ **ID** NO: 5 and a polypeptide of SEQ **ID** NO: 6.

2. The remedial live biotherapeutic formulation for use according to claim 1, further comprising a pharmaceutically acceptable carrier.

3. The remedial live biotherapeutic formulation for use according to claim 1, further comprising an agent adapted to reduce or remove free ammonia from the vaginal environment.

4. The remedial live biotherapeutic formulation according to claim 1 for use in treating bacterial vaginosis, treating pelvic inflammatory disease, treating sexually transmitted infection, treating postpartum endometritis, treating postpartum sepsis, preventing or decreasing the likelihood of preterm birth, preventing or decreasing the likelihood of preterm premature rupture of membranes, preventing or decreasing the likelihood of miscarriage, treating chorioamnionitis, treating intra-amniotic infection, treating infertility, preventing or decreasing the likelihood of ineffectiveness of fertility treatment, treating cervical intraepithelial neoplasia, treating cervical cancer, treating another cancer of the female genitourinary system, decreasing ammonia in the vaginal environment, reducing inflammation, preventing overgrowth of at least one of *Gardnerella vaginalis* and *Prevotella* spp. in the vagina, or a combination thereof.

5. The remedial live biotherapeutic formulation for use according to claim 4, further comprising a pharmaceutically acceptable carrier.

6. The remedial live biotherapeutic formulation for use according to claim 5, further comprising an agent adapted to reduce or remove free ammonia from the vaginal environment.

7. A live biotherapeutic formulation for use in medicine, comprising a selected strain or consortium of strains, wherein the selected strain or consortium of strains consists essentially of any combination of:
(i) *Lactobacillus crispatus* bacteria configured to express at least one of the asparagine synthase B *(asnB)* gene of SEQ ID NO: 2 and the asparagine synthase B *(asnB)* gene of SEQ ID NO: 3; and
(ii) *Lactobacillus crispatus* bacteria containing an asparagine synthase B *(asnB)* gene encoding at least one of a polypeptide of SEQ ID NO: 5 and a polypeptide of SEQ ID NO: 6,
wherein the live biotherapeutic formulation is adapted for administration to the vaginal environment of a female human subject.

8. The live biotherapeutic formulation for use of claim 7, further comprising a redox potential control agent.

9. The live biotherapeutic formulation for use of claim 7, further comprising a pH buffer configured to maintain a healthy pH of the vaginal environment.

10. The live biotherapeutic formulation for use of claim 7, further comprising an antimicrobial agent.

11. The live biotherapeutic formulation for use of claim 7, further comprising a growth promoter.

12. The live biotherapeutic formulation for use of claim 7, further comprising a pharmaceutically acceptable carrier.

## Patentansprüche

1. Lebende biotherapeutische Heilmittelformulierung zur Verwendung in der Medizin, umfassend Bakterien, die angepasst sind, um einen Mangel oder Überschuss an mindestens einem Bakterienstamm in der vaginalen mikrobiellen Umgebung des Subjekts zu beheben, und wobei die Bakterien einen ausgewählten Stamm oder ein Konsortium von Stämmen umfassen, umfassend mindestens eines von Folgenden:
(i) Bakterien *Lactobacillus crispatus,* die konfiguriert sind, um mindestens eines von dem Asparaginsynthase-B(asnB)-Gen von SEQ ID NO: 2 und dem Asparaginsynthase-B*(asnB)*-Gen von SEQ ID NO: 3 zu exprimieren; und
(ii) Bakterien *Lactobacillus crispatus,* die ein Asparaginsynthase-B*(asnB)*-Gen enthalten, das für mindestens eines von einem Polypeptid von SEQ ID NO: 5 und einem Polypeptid von SEQ ID NO: 6 kodiert.

2. Lebende biotherapeutische Heilmittelformulierung zur Verwendung nach Anspruch 1, ferner umfassend einen pharmazeutisch annehmbaren Träger.

3. Lebende biotherapeutische Heilmittelformulierung zur Verwendung nach Anspruch 1, ferner umfassend ein Mittel, das angepasst ist, um freies Ammoniak aus der vaginalen Umgebung zu reduzieren oder zu entfernen.

4. Lebende biotherapeutische Heilmittelformulierung nach Anspruch 1 zur Verwendung beim Behandeln von bakterieller Vaginose, Behandeln von entzündlichen Beckenerkrankungen, Behandeln von sexuell übertragbaren Infektionen, Behandeln von postpartaler Endometritis, Behandeln von postpartaler Sepsis, Verhindern oder Verringern der Wahrscheinlichkeit einer Frühgeburt, Verhindern oder Verringern der Wahrscheinlichkeit eines vorzeitigen Blasensprungs, Verhindern oder Verringern der Wahrscheinlichkeit einer Fehlgeburt, Behandeln von Chorioamnionitis, Behandeln von intra-amniotischen Infektionen, Behandeln von Unfruchtbarkeit, Verhindern oder Verringern der Wahrscheinlichkeit der Unwirksamkeit einer Fruchtbarkeitsbehandlung, Behandeln von zervikaler intraepithelialer Neoplasie, Behandeln von Gebärmutterhalskrebs, Behandeln eines anderen Krebses des weiblichen Urogenitalsystems, Verringern von Ammoniak in der vaginalen Umgebung, Verringern von Entzündungen, Verhindern von übermäßigem Wachstum von mindestens einem von *Gardnerella vaginalis* und *Prevotella* spp. in der Vagina, oder eine Kombination davon.

5. Lebende biotherapeutische Heilmittelformulierung zur Verwendung nach Anspruch 4, ferner umfassend einen pharmazeutisch annehmbaren Träger.

6. Lebende biotherapeutische Heilmittelformulierung zur Verwendung nach Anspruch 5, ferner umfassend ein Mittel, das angepasst ist, um freies Ammoniak aus der vaginalen Umgebung zu reduzieren oder zu entfernen.

7. Lebende biotherapeutische Formulierung zur Verwendung in der Medizin, umfassend einen ausgewählten Stamm oder ein Konsortium von Stämmen, wobei der ausgewählte Stamm oder das Konsortium von Stämmen im Wesentlichen aus einer beliebigen Kombination von Folgendem besteht:
(i) Bakterien *Lactobacillus crispatus,* die konfiguriert sind, um mindestens eines von dem Asparaginsynthase-B(asnB)-Gen von SEQ ID NO: 2 und dem Asparaginsynthase-B*(asnB)*-Gen von SEQ ID NO: 3 zu exprimieren; und
(ii) Bakterien *Lactobacillus crispatus,* die ein Asparaginsynthase-B(asnB)-Gen enthalten, das für mindestens eines von einem Polypeptid von SEQ ID NO: 5 und einem Polypeptid von SEQ ID NO: 6 kodiert,
wobei die lebende biotherapeutische Formulierung zur Verabreichung an die vaginale Umgebung eines weiblichen menschlichen Subjekts angepasst ist.

8. Lebende biotherapeutische Formulierung zur Verwendung nach Anspruch 7, ferner umfassend ein Redoxpotential-Kontrollmittel.

9. Lebende biotherapeutische Formulierung zur Verwendung nach Anspruch 7, ferner umfassend einen pH-Puffer, der konfiguriert ist, um einen gesunden pH der vaginalen Umgebung aufrechtzuerhalten.

10. Lebende biotherapeutische Formulierung zur Verwendung nach Anspruch 7, ferner umfassend ein antimikrobielles Mittel.

11. Lebende biotherapeutische Formulierung zur Verwendung nach Anspruch 7, ferner umfassend einen Wachstumsförderer.

12. Lebende biotherapeutische Formulierung zur Verwendung nach Anspruch 7, ferner umfassend einen pharmazeutisch annehmbaren Träger.

## Revendications

1. Formulation thérapeutique d'agents biothérapeutiques vivants destinée à être utilisée en médecine, comprenant des bactéries adaptées pour remédier à une carence ou à un excès d'au moins une souche bactérienne dans la communauté microbienne vaginale du sujet, et lesdites bactéries comprenant une souche ou un consortium de souches sélectionné comprenant au moins une parmi :
(i) la bactérie *Lactobacillus crispatus* conçue pour exprimer au moins un gène parmi le gène de l'asparagine synthase B *(asnB)* de SEQ ID n° : 2 et le gène de l'asparagine synthase B *(asnB)* de SEQ ID n° : 3 ; et
(ii) la bactérie *Lactobacillus crispatus* contenant un gène de l'asparagine synthase B *(asnB)* codant au moins un polypeptide parmi un polypeptide de SEQ ID n° : 5 et un polypeptide de SEQ ID n° : 6.

2. Formulation thérapeutique d'agents biothérapeutiques vivants destinée à être utilisée selon la revendication 1, comprenant en outre un vecteur pharmaceutiquement acceptable.

3. Formulation thérapeutique d'agents biothérapeutiques vivants destinée à être utilisée selon la revendication 1, comprenant en outre un agent adapté pour réduire ou éliminer l'ammoniaque libre du milieu vaginal.

4. Formulation thérapeutique d'agents biothérapeutiques vivants selon la revendication 1, destinée à être utilisée dans le traitement de la vaginose bactérienne, le traitement d'une maladie inflammatoire pelvienne, le traitement d'une infection sexuellement transmissible, le traitement de l'endométrite post-partum, le traitement de la septicémie post-partum, la prévention ou la diminution de la probabilité d'une naissance avant terme, la prévention ou la diminution de la probabilité d'une rupture prématurée avant terme des membranes, la prévention ou la diminution de la probabilité d'une fausse couche, le traitement de la chorioamnionite, le traitement d'une infection intra-amniotique, le traitement de l'infertilité, la prévention ou la diminution de la probabilité d'inefficacité du traitement de la fertilité, le traitement de la néoplasie intraépithéliale cervicale, le traitement du cancer du col de l'utérus, le traitement d'un autre cancer du système génito-urinaire féminin, la diminution de l'ammoniaque dans le milieu vaginal, la réduction d'une inflammation, la prévention de la prolifération d'au moins une bactérie parmi *Gardnerella vaginalis* et *Prevotella* spp. dans le vagin, ou une combinaison de ceux-ci.

5. Formulation thérapeutique d'agents biothérapeutiques vivants destinée à être utilisée selon la revendication 4, comprenant en outre un vecteur pharmaceutiquement acceptable.

6. Formulation thérapeutique d'agents biothérapeutiques vivants destinée à être utilisée selon la revendication 5, comprenant en outre un agent adapté pour réduire ou éliminer l'ammoniaque libre du milieu vaginal.

7. Formulation d'agents biothérapeutiques vivants destinée à être utilisée en médecine, comprenant une souche ou un consortium de souches sélectionné, ladite souche ou ledit consortium de souches sélectionné consistant essentiellement en une combinaison quelconque de :
(i) la bactérie *Lactobacillus crispatus* conçue pour exprimer au moins un gène parmi le gène de l'asparagine synthase B *(asnB)* de SEQ ID n° : 2 et le gène de l'asparagine synthase B *(asnB)* de SEQ ID n° : 3 ; et
(ii) la bactérie *Lactobacillus crispatus* contenant un gène de l'asparagine synthase B *(asnB)* codant au moins un polypeptide parmi un polypeptide de SEQ ID n° : 5 et un polypeptide de SEQ ID n° : 6,
ladite formulation d'agents biothérapeutiques vivants étant adaptée pour une administration au milieu vaginal d'un sujet humain féminin.

8. Formulation d'agents biothérapeutiques vivants destinée à être utilisée selon la revendication 7, comprenant en outre un agent de contrôle du potentiel redox.

9. Formulation d'agents biothérapeutiques vivants destinée à être utilisée selon la revendication 7, comprenant en outre un tampon de pH conçu pour maintenir un pH sain du milieu vaginal.

10. Formulation d'agents biothérapeutiques vivants destinée à être utilisée selon la revendication 7, comprenant en outre un agent antimicrobien.

11. Formulation d'agents biothérapeutiques vivants destinée à être utilisée selon la revendication 7, comprenant en outre un promoteur de croissance.

12. Formulation d'agents biothérapeutiques vivants destinée à être utilisée selon la revendication 7, comprenant en outre un vecteur pharmaceutiquement acceptable.
